# FASCICULE DE BREVET EUROPEEN

(11) **EP 0 675 895 B1**
(45) Date de publication et mention de la délivrance du brevet: **23.07.1997**
(21) Numéro de dépôt: 94902836.9
(22) Date de dépôt: 20.12.1993
(51) Int. Cl.: C07H 17/04, A61K 31/70

(54) **DERIVES DE L'ETOPOSIDE, LEUR PROCEDE DE PREPARATION, LEUR UTILISATION A TITRE DE MEDICAMENT ET LEUR UTILISATION POUR LA PREPARATION D'UN MEDICAMENT DESTINE AU TRAITEMENT ANTICANCEREUX**
ETOPOSID-DERIVATE, IHRE HERSTELLUNG, IHRE VERWENDUNG ALS MEDIKAMENT UND IHRE VERWENDUNG ZUR HERSTELLUNG EINES ANTIKREBSMITTELS
ETOPOSIDE DERIVATIVES, PROCESS FOR THEIR PREPARATION, THEIR USE AS A DRUG AND IN THE PREPARATION OF A DRUG FOR TREATING CANCER

(30) Priorité: 22.12.1992 FR 9215460
(43) Date de publication de la demande: 11.10.1995
(73) Titulaire: PIERRE FABRE MEDICAMENT, 92100 Boulogne (FR)
(72) Inventeur: ROBIN, Jean-Pierre, SERIPHARM Technopole Univers., F-72000 Le Mans (FR); KISS, Robert, B-1030 Bruxelles (BE); IMBERT, Thierry, F-81290 Viviers-les-Montagnes (FR)
(74) Mandataire: Ahner, Francis
(86) Numéro de dépôt international: FR9301270
(87) Numéro de publication internationale: WO9414829

(56) Documents cités:
- EP-A- 0 415 453
- EP-A- 0 445 021

## Description

La présente invention concerne des dérivés de l'étoposide, leur procédé de préparation et leur utilisation en tant que médicament anti-cancéreux

L'Etoposide a et certains de ses dérivés comme le Téniposide b 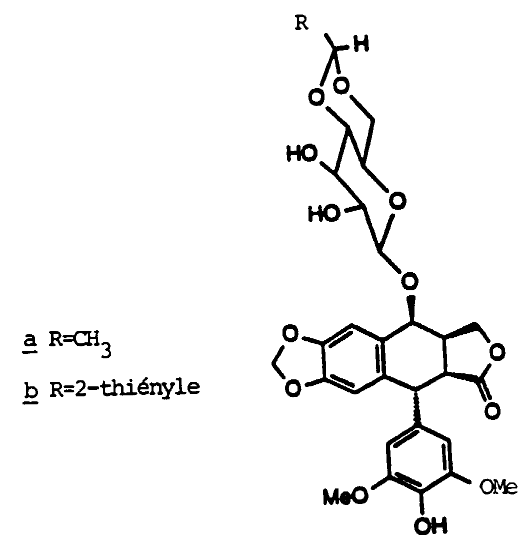 sont connus pour avoir des activités antitumorales utiles en clinique humaine. Ils dérivent d'une part d'un lignage naturel, en particulier de la podophyllotoxine 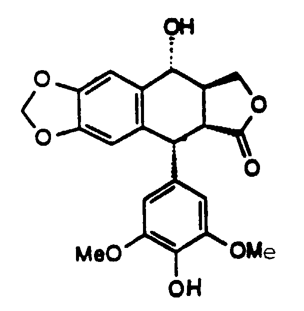 et d'autre part d'une partie glycosylée, en particulier un 4,6-éthylidène β-D-glucopyranose.

La synthèse de l'étoposide a été décrite dans le brevet US-A-3 524 844. Elle a également fait l'objet de nombreuses publications (J. Med. Chem. (1971), 14, 936 ; J. Org. Chem. (1981), 46, 2826 et plus récemment : Heterocycles (1991), 32, 859 ; Tetrahedron (1991), 47, 4675 ; Synthesis (1991), 275).

L'Etoposide et son analogue le Téniposide sont commercialisés comme médicaments pour le traitement d'un certain nombre de tumeurs cancéreuses, ainsi que dans les leucémies aiguës en rechute.

L'inconvénient majeur de ces médicaments, réside dans le fait qu'ils génèrent de nombreux effets secondaires indésirables, tels que la leucopénie ou la thrombopénie.

De nombreux dérivés de l'Etoposide ont été proposés dans la littérature, notamment dans les demandes de brevet EP-A-0 401 800, EP-A-0 461 556, EP-A-0 455 159, EP-A-0 433 678, EP-A-0 358 197, EP-A-0 415 453, EP-A-0 320 988, EP-A-0 423 747, EP-A-0 394 907 ou encore EP-A-0 445 021.

Certains de ces dérivés sont en particulier décrit comme produits intermédiaires de la synthèse de l'Etoposide.

Or, il a été trouvé d'une manière inattendue que certains dérivés de l'Etoposide présentaient une activité anticancéreuse améliorée, en montrant une plus faible toxicité, permettant l'administration de ces produits à titre de médicaments, d'une manière chronique.

La présente invention concerne donc un composé de formule générale I : 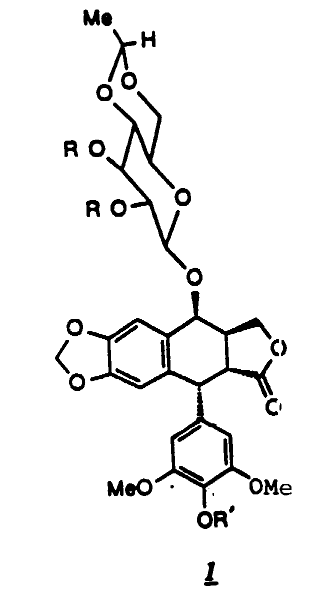 dans laquelle
- R': représente un atome d'hydrogène ou un radical R, et
- R: représente un groupe de formule 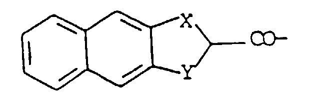 ou un reste acyle de formule

A - Z - CH₂ - CO -
dans lesquelles,
X, Y et Z représentent indépendamment un atome d'oxygène ou un atome de soufre, et
A représente
   - un radical alkyle, linéaire ou ramifié en C₁-C₄,
   - un radical cycloalkyle en C₃-C₆,
   - un radical aryle, en particulier choisi parmi les radicaux phényle, phényle-alkyle, linéraire ou ramifié en C₁-C₄, naphtyle, et ces mêmes radicaux substitués par un à trois substituants, choisis parmi les radicaux hydroxy, alcoxy, linéaire ou ramifié en C₁-C₄ éventuellement perhalogéné avec des atomes de chlore ou de fluor, benzyloxy, phényle, alkyle linéaire ou ramifié en C₁-C₄, et les atomes d'halogène, en particulier le chlore ou le fluor,
   et ses sels physiologiquement acceptables,
   à la condition que lorsque R' représente un radical R, R est choisi parmi les radicaux 4-fluoro-phénoxyacétyle, 4-benzyloxy-phénoxyacétyle, 4-hydroxy-phénoxy-acétyle, 4-méthyl-phénoxy-acétyle, 3,4,5-triméthoxy-phénoxyacétyle, 3,4-méthylène-dioxy-phénoxyacétyle, 2,3-diméthoxy-phénoxyacétyle, 4-trifluoro-méthoxy-phénoxyacétyle, 4-phénylphénoxyacétyle, 1-naphtoxyacétyle, cyclohexylacétyle, 4-méthyl-phénoxyacétyle, 3,4-diméthoxy-phénoxyacétyle.

Par alkyle linéaire ou ramifié en C₁-C₄, on entendra de préférence les radicaux méthyle, éthyle, propyle et butyle, ces mêmes définitions s'appliquant aux restes alkyles des radicaux phényl-alkyle et alcoxy.

D'une manière avantageuse, les composés de formule générale I seront choisis avec R' représentant un atome d'hydrogène.

Pour sa part, R sera choisi de préférence parmi les radicaux éthoxyacétyle, phénoxyacétyle, 4-méthoxyphénoxy-acétyle, 2-méthoxy-phénoxyacétyle, 4-nitro-phénoxyacétyle, 2-nitro-phénoxyacétyle, 2,4-dichloro-phénoxyacétyle, 2,4,5-trichloro-phénoxyacétyle, 2,4,6-trichlorophénoxyacétyle, 4-fluoro-phénoxyacétyle, 2-fluoro-phénoxyacétyle, 4-hydroxy-phénoxyacétyle, 4-benzyloxy-phénoxyacétyle, 4-méthylphénoxy-acétyle, 3,4,5-triméthoxy-phénoxyacétyle, 3,4-méthylènedioxy-phénoxyacétyle, 2,3-diméthoxy-phénoxyacétyle, 4-trifluorométhoxy-phénoxyacétyle, 4-phényl-phénoxyacétyle, 2-naphtoxyacétyle, 1-naphtoxyacétyle, cyclohexylacétyle, 4-méthylphénoxy-acétyle, 3,4-diméthoxy-phénoxyacétyle.

La présente invention concerne également un procédé de préparation d'un composé de formule générale I pour lequel on fait réagir un intermédiaire glycosylé de formule générale 2 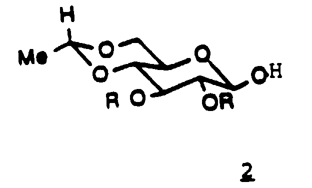 avec un intermédiaire de formule 3 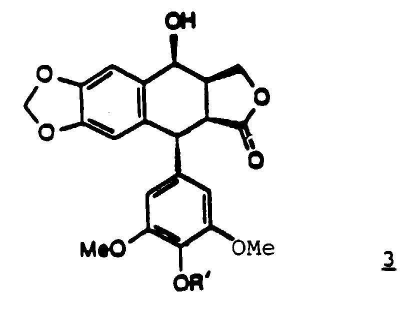 pour lesquelles R et R' sont définis précédemment, et on déprotège éventuellement les fonctions phénol du composé de formule I obtenu.

Le schéma général de synthèse des composés selon l'invention, des intermédiaires de formules 2 et 3 est représenté ci-après, pour R représentant un radical phénoxyacétyle et R' représentant un atome d'hydrogène ou un radical benzyloxycarbonyle.

Bien entendu, la synthèse de l'ensemble des composés de formule I peut se calquer sur celle présentée sur ce schéma.

Pour la lecture de ce schémas, on entendra radical phénoxyacétyle d'une manière générique pour tous les radicaux R définis ci-dessus. On emploiera également Z pour désigner le radical benzyloxycarbonyle.

Le procédé de synthèse utilisé consiste en la préparation de 4,6-éthylidéne-β-D-glucose protégé au niveau de son hydroxy anomère par un benzyloxycarbonyle (Z), puis l'estérification des 2 fonctions alcools en 2 et 3 par des restes phénoxyacétyles pour conduire à des intermédiaires glycosylés protégés, qui sont alors déprotégés par hydrogénation catalytique en présence de Pd/C, de façon conventionnelle.

La préparation de ces dérivés est d'ailleurs décrite dans la demande de brevet EP-A-0 445 021.

Ces intermédiaires sont condensés ensuite à un dérivé de la 4'-déméthyl-épipodo-phyllotoxine protégée au niveau du phénol en 4' par Z, dont la préparation est en particulier décrite dans la demande de brevet EP-A-0 401 800. La condensation s'effectue à basse température aux environs de -20 °C en présence d'un acide de Lewis, comme par exemple l'étherate de BF₃ dans un hydrocarbure chloré, en particulier le chlorure de méthylène, pour conduire aux dérivés de formule générale I pour lesquels R' représente un radical benzyloxycarbonyle.

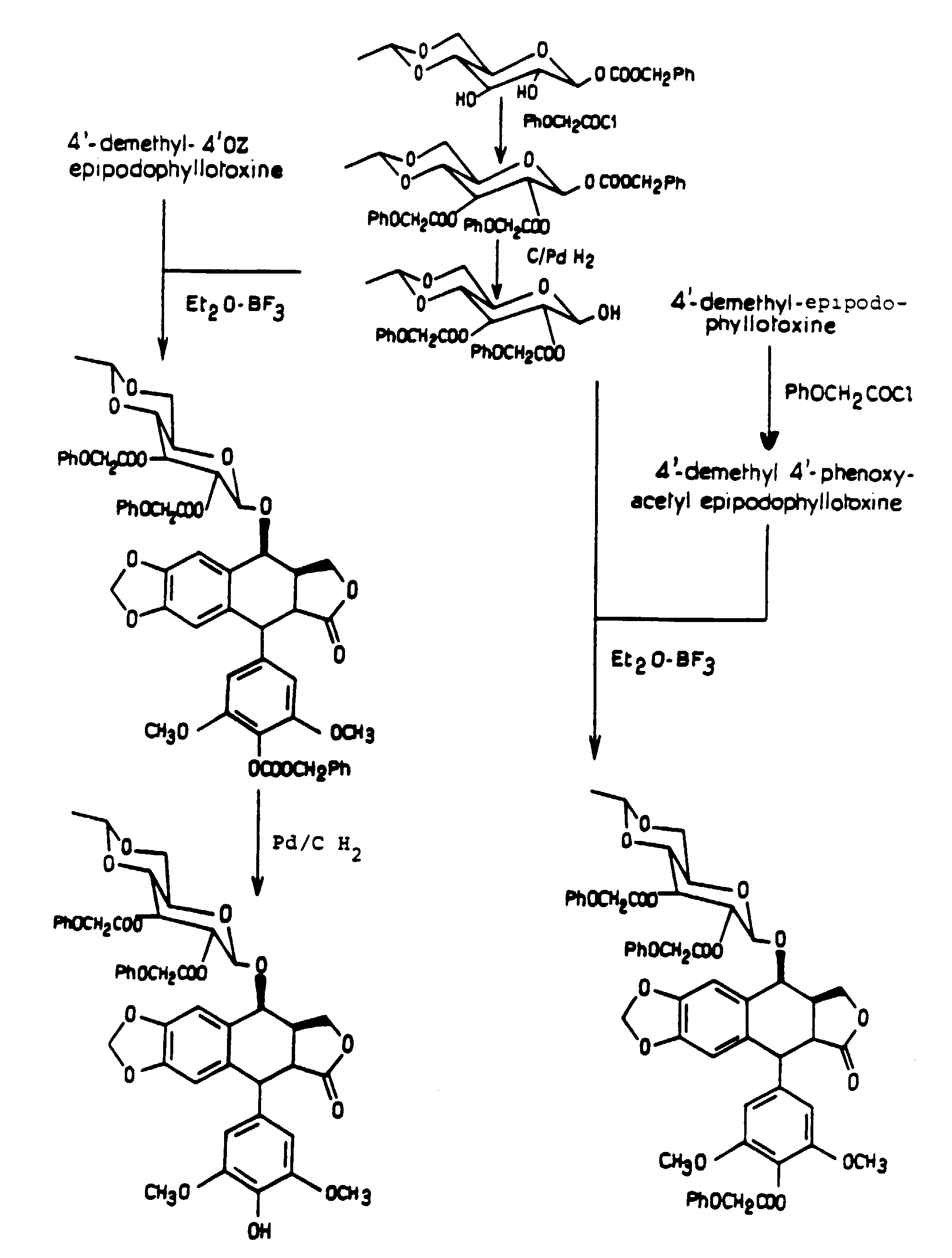

La présence d'une fonction hydroxy anomère sur l'intermédiaire glycosylé, pose toujours le problème de la stéréochimie du produit obtenu, et de son contrôle.

Par le procédé selon la présente invention, on obtient une fraction minime d'anomère α indésirable, permettant d'obtenir directement le composé selon l'invention sans nécessiter une purification ultérieure.

Le dernier stade conduisant au composé selon l'invention pour lequel R' représente un atome d'hydrogène se réalise d'une manière conventionnelle par hydrogénation catalytique en présence de Pd/C, à température ambiante.

Les exemples ci-après de composés de formule générale I permettent d'illustrer la présente invention, sans toutefois chercher à en limiter la portée.

### Exemple 1

### Synthèse du 4'-phénoxyacétyl 4'-déméthyl 4-O-(2,3-bis phénoxyacétyl 4,6-éthylidène glucosyl) épipodophyllotoxine

On a introduit dans du dichlorométhane sec (18 litres), de la (phénoxy-2 acétyl)-4' déméthyl-4' épipodophyl-lotoxine (3 kg, soit 5,62 moles) et du bis (phénoxy-2 acétyl)-2,3 éthylidène-4,6 β-D-glucose (3,2 kg, soit 6,74 moles ou 1,2 éq.). Quand la température a été stabilisée à -18 °C, on a additionné lentement du trifluorure de bore éthérate (1,73 1, soit 14,05 moles, soit 2,5 éq.). La réaction s'est poursuivie à -18 °C pendant 2 h, puis après contrôle par chromatographie en couche mince, on a ajouté de la pyridine (910 ml, soit 2 éq.). La solution a été lavée à l'eau, puis séchée sur MgSO₄ et concentrée sous pression réduite. Le produit brut a été filtré sur gel de silice (70-200 µ ; MeOH (2%)/ CH₂Cl₂) et l'on a obtenu, après évaporation, 4,73 kg d'une poudre blanche, amorphe. Un échantillon a été cristallisé en vue d'une étude analytique. Les résultats sont les suivants :
- Formule brute :: C₅₃H₅₀O₁₉
- Masse molaire :: 990
- Rendement :: 85 %
- PF :: 132-134 °C
[α°]²²D=-67° (c=1 ; CHCl₃)

### Exemple 2

### 4'-(2-méthoxyphénoxyacétyl) 4'-déméthyl 4-O-[2,3-bis(2-méthoxyphénoxyacétyl) 4,6-éthylidène glucosyl] épipodophyllotoxine.

On a repris le mode opératoire de l'exemple 1, mais en utilisant du bis[(méthoxy-2 phénoxy)-2 acétyl)]-2,3 éthylidène-4,6 β-D-glucose et de la [(méthoxy-2 phénoxy)-2 acétyl)]-4' déméthyl-4' épipodophyllotoxine, respectivement, en lieu et place du (phénoxy-2 acétyl)-2,3 éthylidène-4,6 β-D-glucose et de la (phénoxy-2 acétyl)-4' déméthyl-4' épipodophyllotoxine, pour obtenir le produit cherché.
- Formule brute :: C₅₆H₅₆O₂₂
- Masse molaire :: 1080
- Rendement :: 75 %
- PF :: 113-115 °C
[α°]²²D=-52° (c=1 ; CHCl₃)

### Exemple 3

### 4'-(4-méthoxyphénoxyacétyl) 4'-déméthyl 4-O-[2,3-bis(4-méthoxyphénoxyacétyl) 4,6-éthylidène glucosyl] épipodophyllotoxine.

On a repris le mode opératoire de l'exemple 1, mais en utilisant du bis[(méthoxy-4 phénoxy)-2 acétyl)]-2,3 éthylidène 4,6 β-D-glucose et de la [(méthoxy-4 phénoxy)-2 acétyl)]-4' déméthyl-4'-épipodophyllotoxine, respectivement, en lieu et place du (phénoxy-2 acétyl)-2,3 éthylidène-4,6 β-D-glucose, et de la (phénoxy-2 acétyl)-4' déméthyl-4' épipodophyllotoxine pour obtenir le produit cherché.
- Formule brute :: C₅₆H₅₆O₂₂
- Masse molaire :: 1080
- Rendement :: 73 %
- PF :: 107-109 °C
[α°]²²D=-61° (c=1 ; CHCl₃)

### Exemple 4

### 4'-(2-nitrophénoxyacétyl) 4'-déméthyl 4-O-[2,3-bis(2-nitrophénoxy-acétyl)-4,6-éthylidène glucosyl] épipodophyllotoxine.

on a repris le mode opératoire de l'exemple 1, mais en utilisant du bis [(nitro-2 phénoxy)-2 acétyl)]-2,3 éthylidène-4,6 β-D-glucose et de la [(nitro-2 phénoxy)-2 acétyl)]-{4' déméthyl-4' épipodophyllotoxine}, respectivement, en lieu et place du (phénoxy-2 acétyl)-2,3 éthylidène-4,6 β-D-glucose, et de la (phénoxy-2 acétyl)-4'-déméthyl-4' épipodophyllotoxine, pour obtenir le produit cherché.
- Formule brute :: C₅₃H₄₇O₂₅N₃
- Masse molaire :: 1125
- Rendement :: 79 %
- PF :: 135-137 °C

### Exemple 5

### 4'-(4-nitrophénoxyacétyl)-4'-déméthyl-4-O-[2,3-bis(4-nitrophénoxyacétyl)-4,6-éthylidène-glucosyl] épipodophyllotoxine.

On a repris le mode opératoire de l'exemple 1, mais en utilisant du bis[(nitro-4 phénoxy)-2 acétyl)]-2,3 éthylidène-4,6 β-D-glucose et de la [(nitro-4 phénoxy)-2 acétyl)]-4' déméthyl-4' épipodophyllotoxine respectivement, en lieu et place du (phénoxy-2 acétyl)-2,3 éthylidène-4,6 β-D-glucose et de la (phénoxy-2 acétyl)-4' déméthyl-4' épipodophyllotoxine, pour obtenir le produit cherché.
- Formule brute :: C₅₃H₄₇O₂₅N₃
- Masse molaire :: 1125
- Rendement :: 82 %
- PF :: 143-145 °C
[α°]²²D=-72° (c=1 ; CHCl₃)

### Exemple 6

### 4'-(2,4 dichlorophénoxyacétyl) 4'-déméthyl 4-O-[2,3-bis(2,4-dichlorophénoxyacétyl)-4,6-éthylidène glucosyl] épipodophyllotoxine.

On a repris le mode opératoire de l'exemple 1, mais en utilisant du bis[(dichloro-2,4 phénoxy)-2 acétyl)]-2,3 éthylidène-4,6 β-D-glucose et de la [(dichloro-2,4 phénoxy)-2 acétyl)]-4' déméthyl-4' épipodophyllotoxine, respectivement, en lieu et place du (phénoxy-2 acétyl)-2,3 éthylidène-4,6 β-D-glucose, et de la (phénoxy-2 acétyl)-4' déméthyl-4' épipodophyllotoxine, pour obtenir le produit cherché.
- Formule brute :: C₅₃H₄₄O₁₉Cl₆
- Masse molaire :: 1197
- Rendement :: 85 %
- PF :: 115-117 °C
[α°]²²D=-51° (c=1 ; CHCl₃)

### Exemple 7

### 4'-(2,4,5-trichlorophénoxyacétyl) 4'-déméthyl 4-O-[2,3-bis(2,4,5-trichlorophénoxyacétyl) 4,6-éthylidène glucosyl] épipodophyllotoxine.

On a repris le mode opératoire de l'exemple 1, mais en utilisant du bis[(trichloro-2,4,5 phénoxy)-2 acétyl)]-2,3 éthylidène-4,6 β-D-glucose et de la [(trichloro-2,4,5 phénoxy)-2 acétyl)]-4' déméthyl-4' épipodophyllotoxine, respectivement, en lieu et place du (phénoxy-2 acétyl)-2,3 éthylidène-4,6 β-D-glucose, et de la (phénoxy-2 acéthyl)-4' déméthyl-4' épipodophyllotoxine, pour obtenir le produit cherché.
- Formule brute :: C₅₃H₄₁O₁₉Cl₉
- Masse molaire :: 1300,5
- Rendement :: 75 %
- PF :: 126-128 °C
[α°]²²D=-43° (c=1 ; CHCl₃)

### Exemple 8

### 4'-(2,4,6-trichlorophénoxyacétyl) 4'-déméthyl 4-O-[2,3-bis(2,4,6-trichlorophénoxyacétyl)-4,6-éthylidène glucosyl]-épipodophyllotoxine.

On a repris le mode opératoire de l'exemple 1, mais en utilisant du bis (trichloro-2,4,6 phénoxy)-2 acétyl)]-2,3 éthylidène-4,6 β-D-glucose et de la [(trichloro-2,4,6 phénoxy)-2 acétyl)]-4' déméthyl-4' épipodophyllotoxine, respectivement, en lieu et place du (phénoxy-2 acétyl)-2,3 éthylidène-4,6 β-D-glucose, et de la (phénoxy-2 acétyl)-4' déméthyl-4' épipodophyllotoxine, pour obtenir le produit cherché.
- Formule brute :: C₅₃H₄₁O₁₉Cl₉
- Masse molaire :: 1300,5
- Rendement :: 80 %
- PF :: 120-122 °C
[α°]²²D=-10° (c=1 ; CHCl₃)

### Exemple 9

### 4'-(2-fluorophénoxyacétyl) 4'-déméthyl 4-O-[2,3-bis(2-fluorophénoxyacétyl)-4-éthylidène glucosyl]-épipodophyllotoxine.

On a repris le mode opératoire de l'exemple 1, mais en utilisant du bis[(fluoro-2 phénoxy)-2 acétyl)]-2,3 éthylidène-4,6 β-D-glucose et de la [(fluoro-2 phénoxy)-2 acétyl)]-4' déméthyl-4' épipodophyllotoxine, respectivement, en lieu et place du (phénoxy-2 acétyl)-2,3 éthylidène-4, 6 β-D-glucose et de la (phénoxy-2 acétyl)-4' déméthyl-4' épipodophyllotoxine, pour obtenir le produit cherché.
- Formule brute :: C₅₃H₄₇O₁₉F₃
- Masse molaire :: 1140
- Rendement :: 57 %
- PF :: amorphe
[α°]²²D=-58° (c=1 ; CHCl₃)

### Exemple 10

### 4'-éthoxyacétyl 4'-déméthyl 4-O-(2,3-bis éthoxy acétyl-4,6-éthylidène-glucosyl)-épipodophyllotoxine.

On a repris le mode opératoire de l'exemple 1, mais en utilisant du bis(éthoxy-2 acétyl)-2,3 éthylidène-4,6 β-D-glucose et de l'(éthoxy-2 acétyl)4' déméthyl-4' épipodophyllotoxine, respectivement, en lieu et place du (phénoxy-2 acétyl)-2,3 éthylidène-4, β-D-glucose, et de la (phénoxy-2 acétyl)-4' épipodophyllotoxine, pour obtenir le produit cherché.
- Formule brute :: C₄₁H₅₀O₁₉
- Masse molaire :: 846
- Rendement :: 71 %
- PF :: 195-198 °C
[α°]²²D=-64° (c=1 ; CHCl₃)

### Exemple 11

### 4'-[2,2-(2,3-naphtylidène-dioxy)-acétyl]-4'-déméthyl-4-O-[2,3-bis[2,2(2,3-naphtylidènedioxy)-acétyl]-4,6-éthylidène-glucosyl) épipodophyllotoxine.

On a repris le mode opératoire de l'exemple 1, mais en utilisant du bis(naphtylidendioxy-2,2 acétyl)-2,3 éthylidène-4, β-D-glucose et de la (naphtylidendioxy-2,2 acétyl)-4' épipodophyllotoxine, respectivement, en lieu et place du (phénoxy-2 acétyl)-2,3 éthylidène-4,6 β-D-glucose et de la (phénoxy-2 acétyl)-4' déméthyl-4' épipodophyllotoxine, pour obtenir le produit cherché.
- Formule brute :: C₆₅H₅₀O₂₂
- Masse molaire :: 1182
- Rendement :: 64 %
- PF :: 157-159 °C
[α°]²²D=-40° (c=1 ; CHCl₃)

### Exemple 12

### 4'-benzylthioacétyl-4'-déméthyl-4-O-[2,3-bis-benzylthioacétyl-4,6-éthylidène-glucosyl]épipodophyllotoxine.

On a repris le mode opératoire de l'exemple 1, mais en utilisant du bis(benzylthio-2 acétyl)-2,3 éthylidène-4,6 β-D-glucose et de la (benzylthio-2 acétyl)-4' déméthyl-4' épipodophyllotoxine, respectivement, en lieu et place du (phénoxy-2 acétyl)-2,3 éthylidène-4,6 β-D-glucose, et de la (phénoxy-2 acétyl)-4' déméthyl-4' épipodophyllotixine, pour obtenir le produit cherché.
- Formule brute :: C₅₆H₅₆O₁₆S₃
- Masse molaire :: 1080
- Rendement :: 49 %
- PF :: amorphe
[α°]²²D=-48° (c=1 ; CHCl₃)

### Exemple 13

### 4'-phénylthioacétyl-4'-déméthyl-4-O-[2,3-bis phénylthioacétyl-4,6-éthylidéne-glucosyl] épipodophyllotoxine.

On a repris le mode opératoire de l'exemple 1, mais en utilisant du bis(phénylthio-2 acétyl)-2,3 éthylidène-4,6 β-D-glucose et de la (phénylthio-2 acétyl) -4' déméthyl-4' épipodophyllotoxine, respectivement, en lieu et place du (phénoxy-2 acétyl)-2,3 éthylidène-4, β-D-glucose, et de la (phénoxy-2 acétyl)-4' déméthyl-4' épipodophyllotoxine, pour obtenir le produit cherché.
- Formule brute :: C₅₃H₅₀O₁₆S₃
- Masse molaire :: 1038
- Rendement :: 82 %
- PF :: amorphe
[α°]²²D=-60° (c=1 ; CHCl₃)

### Exemple 14

### 4'-(4-fluorophénoxyacétyl)-4'-déméthyl 4-O-[2,3-bis(4-fluorophénoxyacétyl)-4,-éthylidène-glucosyl]-épipodphyllotoxine.

On a repris le mode opératoire de l'exemple 1, mais en utilisant du 2,3-bis(4-fluorophénoxy acétyl) 4,-éthylidène β-D-glucose et de la 4'-(4-fluorophénoxy acétyl) 4'-déméthyl épipodophyl-lotoxine, pour obtenir le produit recherché isolé par chromatographie sur colonne de silice (élution CH₂Cl₃-MeOH 99/1) en tant que poudre amorphe.
IR (Nujol, v(cm⁻¹)) : 1755, 1590, 1480, 1150, 1080, 820, 720.
RMN-¹H(400 MHz, CDCl₃) δ, ppm : 6,9 (m,8H,Ar) ; 6,79 (m,2H,Ar) ; 6,77 (s,1H,H-5) ; 6,67 (m,2H,Ar) ; 6,51 (s,1H,H-8) ; 6,26 (s,2H,H-2',H-6) ; 5,92 (d,1H,J.1,0Hz,OCHAO); 5,71 (d,1H,J=0,9Hz,OCHBO); 5,34 (t,1H,J3-2-9,4Hz,H-3"); 5,03 (dd,1H,J2-1=7,9Hz,J2-3=9,2Hz,H-2"); 4,91 (d,1H,J1-2=7,8Hz,H-1") ; 4,86 (s,2H,4'-ArOCH₂) ; 4,83 (m,1H,J=3,5Hz,J=3,OHz,H-4); 4,69 (q,1H,J7-CH₃-4,9Hz,H-7") ; 4,58 (d,1H,J1-2=5,3Hz,H-1) ; 4,56 (7AB,2H,J=16,5Hz,J6,6Hz,ArOCH₂); 4,39 (dd,1H,J=8,9Hz,J=10,5Hz,H-3a(A)); 4,37 (dAB,1H,J-16,4Hz,ArOCHA) ; 4,22 (t,1H,J=8,20Hz,H-3a(B)); 4,20 (m,1H,H-6"B) ; 4,15 (dAB,1H,J=16,3Hz,ArOCHB-) ; 3,66 (s,6H,OCH₃) ; 3,59 (t,1H,JAB=10,2Hz,H-6"B) ; 3,52 (t,1H,J4"-5"=9,45Hz,H-4"); 3,42 (ddd,1H,J5"-6"=4,8Hz,J5"-4"=9,45Hz,H-5"); 3,15 (dd,1H,J2-3=14,1Hz, J2-1=5,20Hz,H-2) ; 2,85 (m,1H,H-3) ; 1,35 (d,3H,JCH₃-7"=5,0Hz,CH₃).

| Anal. C₅₃H₄₇F₃O₁₉ | | |
|---|---|---|
| | C | H |
| Calc. % | 60,92 | 4,53 |
| Tr. % | 59,34 | 4,42 |

### Exemple 15

### 4'-(4-benzyloxy(phénoxyacétyl)-4'-déméthyl-4-O-[2,3-bis(4-benzyloxyphénoxyacétyl)-4,6-éthylidène-glucosyl]-épipodophyllotoxine.

On a repris le mode opératoire de l'exemple 1, mais en utilisant du 2,3-bis(4-benzyloxy phénoxyacétyl)-4,6-éthylidène-β-D-glucose et de la 4'-(4-benzyloxyphénoxyacétyl)-4'-déméthylépipodophyllotoxine issu de manière analogue à l'exemple 14 pour obtenir le produit recherché isolé par chromatographie en tant que poudre amorphe.
IR (Nujol, v cm⁻¹) : 1760, 1690, 1590, 1490, 1200, 1055, 710.
RMN-¹H (400 MHz, CDCl₃) δ ppm : 7,45-7,22 (m,15H,Ph) ; 6,92-6,70 (m,10H) ; 6,68 (1,1H,H-5) ; 6,50 (d,2H,J=9,1Hz) ; 6,38 (s,1H,H-8) ; 6,18(s,2H,H-2', H-6') ; 5,80 (s,1H,OCHAO) ; 5,55 (d,1H,J-0,9Hz,OCHBO); 5,29 (t,1H,J=9,45Hz,H-3") ; 4,97 (dd,1H,J=9,45Hz,H-3") ; 4,97 (dd,1H,J=7,93Hz,J=9Hz,H-2"); 4,94 (s,2H, PhCH₂O-4') ; 4,84 (d,1H,J=5,5Hz,H=1") ; 4,84 (s,2H,PNCH₂O) ; 4,80 (qAB,2H,J=11,5Hz, PhCH₂O) ; 4,77 (s,2H,ArOCH₂-4') ; 4,74 (d,1H,J4-3=3,5Hz,H-4); 4,61 (q,1H,J7"-CH₃=5,0Hz,H-7") : 4,48 (d,1H,J1-2=6Hz,H-1); 4,47 (qAB,2H,J-16,5Hz,J=3,24Hz,ArOCH₂CO) ; 4,32 (t,1H,J=10,9Hz,J=8,6Hz,H-3a(A)) ; 4,30 (dAB,1H,J=16,5Hz,ArOCHβCO) ; 4,15 (t,1H,J=8,7Hz,H-3a(B)) ; 4,13 (dd,1H,JAB=10,26Hz,J6"-5"=4,7Hz,H-6"A) ; 4,04 (dAB,1H,J=16,4Hz,ArOCHACO); 3,56 (s,6H,OCH₃) ; 3,51 (t,1H,JAB=10,2Hz,H-6"B) ; 3,44 (t,1H,J4"-5"=9,4Hz,H-4"); 3,35(ddd,1H,J5"-4"=9,66Hz,J5"-6"=4,7Hz,H-5"); 3,08 (dd,1H,J2-3=14,1Hz,J2-1=5,2Hz,H-2) ; 2,75 (m,1H,H-3) ; 1,26 (d,3H,JCH₃-7"=5,OHz,CH₃).

### Exemple 16

### 4'-(4-hydroxyphénoxyacétyl)-4'-déméthyl-4-O-[2,3-bis(4-hydroxyphénoxyacétyl)-4,6-éthylidèneglucosyl]-épipodophyllotoxine.

2,27 g de 4'-(4-benzyloxyphénoxyacétyl)-4'-déméthyl 4-O-[2,3-bis(4-benzyloxyphénoxyacétyl)-4,6-éthylidène]-épipodophyllotoxine issu de l'exemple 15 dissous dans 25 ml d'acétate d'éthyle sont hydrogénés en présence de 0,9 g de Pd/C à 10 % sous 50 psi pendant 8 h. Le milieu est filtré et évaporé sous pression réduite. Le produit de débenzylation est ensuite chromatographié sur colonne de silice avec un éluant de CH₂Cl₂/MeOH : 97/3. 1,40 g sont obtenus soit un rendement de 80 %. F = 163 °C.
IR (Nujol, v(cm⁻¹)) : 3360, 1745, 1500, 1200, 1070, 820, 710.
RMN⁻¹H (400 MHz, CD₃COCD₃) δ ppm : 8,0 (s,1H,OH) ; 7,96 (s,1H,OH) ; 7,95 (s,1H,OH) ; 7,09 (s,1H,H-5) ; 6,86 (d,2H,J=9,OHz,Ar) ; 6,77 (d,2H,J=9,0Hz,Ar) ; 6,74 (m,6H,Ar) ; 6,59 (d,2H,J=8,9Hz,Ar) ; 6,55 (s,1H,H-8) ; 6,40 (s,2H,H-2',H-6') ; 5,98 (d,1H,J=0,8Hz,OCHAO); 5,84 (s,1H,OCHBO) ; 5,40 (t,1H,J3"-2"=9,2Hz,H-3") ; 5,35 (d,1H,J=7,9Hz,H-1"); 5,09 (d,1H,J4-3-3,3Hz,H-4); 5,0 (dd,1H,J2"-1"=7,9Hz,J2"-3"=9,4Hz,H-2"); 4,85 (s,2H,ArOCH₂CO-4') ; 4,80 (q,1H,J7"-CH₃=5,0Hz,H-7") : 4,63 (d,1H,J1-2=5,3Hz,H-1) ; 4,59 (s,2H,ArOCH₂CO) ; 4,43 (dAB,1H,J=16,5Hz,ArOCHACO) ; 4,37 (dd,1H,J=10,1Hz,J=8,7Hz,H-3a(A)); 4,27 (dd,1H,J=8,3Hz,J=7,6Hz,H-3a(B)) ; 4,21 (m,1H,H-6"A) ; 4,06 (dAB,1H,J=16,6Hz,ArOCHBCO) ; 3,67 (m,OCH₃,H-6"B,H-4") ; 3,57 (m,1H,H-5") ; 3,19 (dd,1H,J2-3=14,3Hz,J2-1=5,3Hz,H-2); 3,07 (m,1H,H-3) ; 1,26(d,3H,JCH₃-7"=5,06Hz,CH₃).

| Anal. C₅₂H₅₀O₂₂ | | |
|---|---|---|
| | C | H |
| Calc. % | 60,82 | 4,91 |
| Tr. % | 60,78 | 4,96 |

### Exemple 17

### 4'-déméthyl 4-O-(2,3-bis-phénoxy-acétyl-4,6-éthylidène glucosyl)-épipodophyllotoxine.

### 1er stade : Condensation de la partie glycosylée avec la partie épipodophyllotoxine protégée en 4'.

### Synthèse du 4'-benzyloxycarbonyl-4'-déméthyl-4-O-(2,3-bis-phénoxyacétyl-4,6-éthylidène glucosyl)-épipodophyllotoxine.

On additionne en 30 mn de l'éthérate de BF₃ (2,9 ml, 2,5 éq.) à une solution agitée à -18 °C, et maintenu sous azote, de 5 g (9,35 mmoles) de 4'-benzyloxycarbonyl-4'-déméthyl-épipodophyllotoxine et de 2,3-bis phénoxyacétyl-4,6-éthylidéne β-D-glucose (6,65 g, 1,5 éq., 14 mmoles) dans 50 ml de CH₂Cl₂ anhydre. Après 2 h d'agitation à -18 °C, la réaction est stoppée par addition de 3 ml de pyridine, et la phase organique est lavée a l'eau, séchée sur sulfate de magnésium et concentrée sous pression réduite. Après chromatographie sur gel de silice (éluant CH₂Cl₂), on obtient 8,13 g du dérivé glucosylé à l'état amorphe (Rdt = 88 %).

### 2ème stade : Hydrogénolyse de la position 4'.

Dans une bouteille à hydrogéner, on place 5 g de dérivé glucosylé obtenu au premier stade dans 50 ml d'acétone anhydre, en présence de 500 mg de Pd/C à 10 %. Après avoir fermement relié la bouteille à une arrivée d'hydrogène, la solution est agitée magnétiquement et l'hydrogène est introduit avec une surpression de 0,1013 bar (0,1 atmosphère), l'agitation est maintenue pendant 2 h. Après élimination du catalyseur par filtration et évaporation du solvant sous pression réduite, on obtient 4,32 g (Rdt quantitatif) de produit à l'état amorphe.

L'étude analytique a donné les résultats suivants :
IR (Nujol) ( , cm⁻¹) : 1760 (c=o), 1600, 1050.
RMN⁻¹H (400 MHz, CDCl₃) δ ppm : 6,7 - 7,3 (8H, m, Ar) ; 6,77 (1H, s, H-5) ; 6,73 (2H, d, H Ar) ; 6,49 (1H, s, H-8) ; 6,24 (2H, s, H-2',6') ; 5,9 et 5,64 (2H,2d,OCH₂O) ; 5,43 (1H,s,OH phénol) ; 5,37 (1H,t,J=9,5Hz,H-3"); 5,05 (1H,dd,H-2") ; 4,93 (1H,d,J=7,9Hz,H-1") ; 4,82 (1H,d,J=3,4Hz,H-4) ; 4,69 (1H,g,H-7") ; 4,60 (2H,m,OCH₂CO) ; 4,55 (1H,H-1) ; 4,40 et 4,18 (2H,2d,OCH₂CO) ; 4,39 (1H,dd,H-3a(A)) ; 4,18 (2H,m,H-3a (B) et H-6"éq.) ; 3,75 (6H,s,OCH₃) ; 3,59 (1H,t,H-6"ax) ; 3,52 (1H,m,H-4") ; 3,44 (1H,m,H-5") ; 3,15 (1H,dd,J2-1=5,2Hz,J2-3=14,1Hz,H-2) ; 2,87 (1H,m,H-3) ; 1,35 (1H,d,J=5,1Hz).

### Exemple 18

### 4'-déméthyl-4-O-[2,3-bis(4-méthoxyphénoxy acétyl)-4,6-éthylidéneglucosyl]-épipodophyllotoxine.

1er stade : On a repris le mode opératoire de l'exemple 17, mais en utilisant le 2,3-bis(4-méthoxyphénoxyacétyl) -4,6-éthylidéne-β-D-glucose et le 4'-benzyloxycarbonyl-4'-déméthyl-épipodophyllotoxine pour obtenir le produit intermédiaire désiré 4'-benzyloxycarbonyl 4'-déméthyl 4-0-[2,3-bis(4-méthoxyphénoxyacétyl)4,-éthylidène-glucosyl)-épipodophyllotoxine dont les caractéristiques sont les suivantes : Amorphe ; Rdt. 100 %.
IR (Nujol) (v, cm⁻¹) 1750, 1590, 1220, 1020.
RMN-¹H (400 MHz, CDCl₃) , δ, ppm : 7,32-7,42 (m,5H,Ph) ; 6,86 (m,8H,ArO-5) ; 6,66 (d,2H,Ar) ; 6,48 (s,1H,H-8) ; 6,25 (s,2H,H-2',H-6') ; 5,90 (s,1H,OCHAO) ; 5,67 (s,1H,OCHBO) ; 5,35 (t,1H,J=9,4Hz,H-3") ; 5,24 (s,2H, PhCH₂) ; 5,04 (dd,1H,J=7,9Hz J=9,2Hz,H-2") ; 4,91 (d,1h,J=7,8Hz,H-1") ; 4,81 (d,1H,J=3,4Hz,H-4); 4,68 (q,1H,J=4,9Hz,H-7") ; 4,55 (d,1H,J=5,15Hz,H-1); 4,54 (qAB,2H,J=4,15Hz,J=16,5Hz,ArOCH₂) ; 4,38 (dd,1H,J=10,2Hz,J=9,2Hz,H-3a (A)) ; 4,36 (d,1H,J=16,4Hz,ArOCHA) ; 4,20 (m,2H,H-6"A,H3a(B)); 4,11 (d,1H,J=16,4Hz,ArOCHB) ; 3,71 (s,3H,CH₃OPh) ; 3,68 (s,3H,CH₃OPh) ; 3,65 (s,6H,OCH₃) ; 3,58 (t,1H,J=10,2Hz,H-6"B) ; 3,51 (t,1H,J=9,4Hz,H-4") ; 3,42 (ddd,1H,J5"-4"=9,5Hz,J5"-6"=5,0Hz,H-5") ; 3,15 (dd,1H,J=5,2Hz,J=14,2Hz,H-2); 2,83 (m,1H,H-3) ; 1,34 (d,3H,J=4,95Hz,CH₃).

2ème stade : Hydrogénolyse de la position 4'

Cet intermédiaire protégé en 4' (1 g) est soumis à une hydrogénolyse dans 30 ml d'un mélange 4:1 d'éthanol : acétone contenant 0,18 g de Pd/C à 10 % sous atmosphère d'hydrogène à température et pression ambiante sous bonne agitation, pendant 2 h. Après contrôle en CCM (éluant CH₂Cl₂/MeOH 97/3), le catalyseur est filtré sur verre fritté et lavé avec de l'éthanol. Le filtrat est évaporé sous pression réduite pour fournir le composé dont les caractéristiques physiques sont les suivantes :
Cristaux F = 196 °C ; Rdt = 100 % ;
IR (Nujol ; v, cm⁻¹) ; 3400, 1750, 1590, 700.
RMN 1H, 400 MHz, CDCl₃ (δ, ppm) : 6,79 (m, 5H,ArO,H-5) ; 6,76(d,2H,J=7,67Hz,J=9,0Hz,ArO); 6,67(d,2H,J=9,0Hz,ArO) ; 6,49 (s,1H,H-8) ; 6,23 (s,2H,H-2',H-6') ; 5,92 (d,1H,J=1,1Hz,OCHAO) ; 5,69 (d,1H,J=1,1Hz,OCHBO); 5,41 (s,1H, OH) ; 5,36 (t,1H,J3"-2"=9,4Hz,H-3"); 5,05 (dd,1H,J2"-1"=7,92Hz,J2"-3"=9,25Hz,H-2") ; 4,91 (d,1H,J1"-2"=7,82Hz,H-1") ; 4,82 (d,lH,J4-3=3,4Hz,H-4) ; 4,69 (q,1H,J=5,0Hz,H-7"); 4,54 (qAB,2H,J=16,4Hz,J=4,3z,ArOCH₂) ; 4,53 (d,1H,H-1) ; 4,38 (dd,1H,J=8,84Hz,J=10,6Hz,H-3a(A)); 4,37 (d,1H,J=16,4Hz,ArOCHA) ; 4,20 (m,2H,H-6"A,H-3a(B); 4,12 (d,1H,J=16,4Hz,ArOCHB); 3,75 (s,6H,OCH₃) ; 3,71 (s,3H,CH₃O-PhOCH₂) ; 3,68 (s3H,CH₃O-PhOCH₂) ; 3,59 (t,1H,J=10,1Hz,H-6"B); 3,51 (t,1H,J-9,5Hz,H-4"); 3,42 (ddd,1H,J=4,7Hz,J=9,6Hz,H-5") ; 3,13 (dd,1H,J2-1=5,2Hz,J2-3=14,1Hz,H-2); 2,87 (m,1H,H-3) ; 1,34(d,3H,J=4,98Hz,CH₃).

| Anal. C₄₇H₄₈O₁₉ | | |
|---|---|---|
| | C | H |
| Calc. % | 61,56 | 5,27 |
| Tr. % | 61,40 | 5,19 |

### Exemple 19

### 4'-déméthyl-4-O-[2,3-bis(4-fluoro-phénoxy acétyl)-4,6-éthylidène-glucosyl] épipodophyllotoxine.

1er stade : On a repris le mode opératoire de l'exemple 17, mais en utilisant le 2,3-bis(4-fluorophénoxyacétyl) 4,6-éthylidène-β-D-glucose et le 4'-benzyloxy carbonyl-4'-déméthyl-épipodophyllotoxine pour obtenir l'intermédiaire désiré : 4'-benzyloxycarbonyl-4'-déméthyl-4-0-[2,3-(4-fluorophénoxyacétyl)-4,6-éthylidène-glucosyl]épipodophyllotoxine dont les caractéristiques sont les suivantes :
Amorphe, Rdt. : 98 % ; IR (Nujol, v, cm⁻¹) : 1750, 1590, 1180, 705.
RMN 1H (400 MHz, CDCl₃), (δ, ppm) : 7,42-7,32 (m,5H,Ph) ; 6,93 (m,4H,F-Ph) ; 6,80 (m,2H,F-Ph) ; 6,77 (s,1H,H-5) ; 6,67 (m,2H,F-Ph) ; 6,51 (s,1H,H-8) ; 6,25 (s,2h,H-2 ',H-6') ; 5,92 (d,1H,J=1,12Hz,OCHAO); 5,71 (d,1H,J=0,92Hz,OCHBO) ; 5,34 (t,1H,J3"-2"=9,4Hz,H-3") ; 5,25 (s,2H, PhCH₂) ; 5,03 (dd,1H,J2"-1"=7,9Hz,J2"-3"=9,2Hz,H-2") ; 4,91 (d,1H,J1"-2"=7,8Hz,H-1"); 4,83 (d,1H,J=3,4Hz,H-4) ; 4,69 (q,1H,J=5,0Hz,H-7") ; 4,57 (d,1H,H-1) ; 4,55 (qAB,2H,J=16,5Hz,J=67Hz,ArOCH₂) ; 4,39 (dd,1H,J=10,5Hz,J=8,7Hz,H-3a(A)) ; 4,37 (dAB,1H,J=16,3Hz,ArOCHA); 4,21 (m,2H,H-6"A,H-3a(B)) ; 4,15 (dAB,1H,J=16,4Hz,ArOCHB) ; 3,66 (s,6H,OCH₃) ; 3,59 (t,1H,J=1 0,2Hz,H-6"B) ; 3,52 (t,1H,J4"-5"=9,45Hz,H-4"); 3,42 (ddd,1H,J5"-4"=9,54Hz,J5"-6"=4,8Hz,H-5"); 3,15 (dd,1H,J=14,1Hz,J2-1=5,2Hz,H-2); 2,85 (m,1H,H-3) ; 1,35 (d,3H,J=5,OHz,CH₃).

2ème stade : Hydrogénolyse de la position 4'

Cet intermédiaire protégé en 4' est soumis à une hydrogénolyse sur Pd/C comme décrit dans l'exemple 18, pour conduire au composé dont les caractéristiques physiques sont les suivantes :
Cristaux F = 164 °C , Rdt = 94 % ;
IR (Nujol, v(cm⁻¹)) : 3400, 1755, 1595, 1170.
RMN 1H, 400 MHz, CDCl₃, (δ, ppm) : 6,93 (m,4H,ArO) ; 6,79 (m,2H,ArO) ; 6,76 (s,lH,H-5) ; 6,66 (m,2H,ArO) ; 6,51 (s,1H,H-8) ; 6,23 (s,2H,H-2',H-6') ; 5,92 (d,1h,J=0,97Hz,OCHAO); 5,71 (d,1H,J=0,98Hz,OCHBO) ; 5,41 (s,1H,OH) ; 5,34 (t,1H,J3"-2"=9,4Hz,H-3"); 5,03 (dd,1H,J2"-1"=7,9Hz,JZ"-3"=9,1Hz,H-2") ; 4,92 (d,1H,J1"-2"=7,8Hz,H-1") ; 4,83 (d,1H,J4-3=3,4Hz,H-4) ; 4,69 (q,1H,J7"-CH₃-4,9Hz,H-7"); 4,56 (qAB,2H,J-6,6Hz,J=16,5Hz,ArOCH₂) ; 4,54 (d,1H,J1-2=4,86Hz,H-1) ; 4,38 (t,1H,J=8,9Hz,J=10,3Hz,H-3a(A)); 4,375 (d,1H,J=16,5Hz,ArOCHA-); 4,21 (m,2H,H-3a(B),H-6"A); 4,15 (d,1H,J=16,4Hz,ArOCHB); 3,76 (s,6H,OCH₃) ; 3,59 (t,1H,JAB=10,1Hz,H-6"B); 3,52 (t,1H,J4"-3"=9,4Hz,H-4") ; 3,42 (ddd,1h,J5"-6"=4,75Hz,J5"-4"=9,6Hz,H-5") 3,12 (dd,1h,J2-3=14,1Hz,J2-1=5,2Hz,H-2); 2,87 (m,1H,H-3) ; 1,35 (d,3H,JCH₃-7"=4,98Hz,CH₃).

| Anal. C₄₅H₄₂F₂O₁₇ | | |
|---|---|---|
| | C | H |
| Calc. % | 60,54 | 4,74 |
| Tr. % | 60,55 | 4,73 |

### Exemple 20

### 4'-déméthyl-4-O-[2,3-bis(4-hydroxyphénoxy acétyl)-4,6-éthylidène-glucosyl]épipodophyllotoxine.

1er stade : Condensation de la partie glucosylée et de la partie épipodophyllotoxine.

On a repris le mode opératoire de l'exemple 17, mais en utilisant le 2,3-bis(4-benzyloxyphénoxy acétyl)-4,6-éthylidène-β-D-glucose et le 4'-benzyloxy carbonyl-4'-déméthylépipodophyllotoxine pour obtenir l'intermédiaire désiré : 4'-benzyloxycarbonyl-4'-déméthyl-4-O-[2,3-bis(4-benzyloxyphénoxyacétyl)-4,6-éthylidène-glucosyl]-épipodophyllotoxine dont les caractéristiques sont les suivantes :
Amorphe, Rdt = 98 % ;
IR (Nujol, v (cm⁻¹)) : 1750, 1590, 1490, 1200, 1070, 810, 715.
RMN-¹H (400MHz, CDCl₃) δ ppm : 7,44-7,20 (m,Ph) ; 6,85-6,70 (m,OArO) ; 6,68 (s,1H,H-5) ; 6,59 (d,2H,J=9,1Hz,OArO) ; 6,38 (s,1H,H-8) ; 6,18 (s,2H,H-2', H-6') ; 5,79 (s,1H,OCHAO) ; 5,55 (s,1H,OCHBO) ; 5,28 (t,1H,J=9,45Hz,H-3") ; 5,17 (s,2H,PhCH₂) ; 4,97 (dd,1H,J2"-1"=7,9Hz,J2"-3"=9Hz,H-2") ; 4,84 (d,1H,J1"-2"=7Hz,H-1"); 4,83 (s,2H,PhCH₂) : 4,79 (qAB,2H,J-5,1Hz,J=11,6Hz, PhCH₂OArO) ; 4,74 (d,1H,J4-3=3,4Hz,H-4) ; 4,61 (q,1H,J=5,0Hz,H-7") ; 4,50 (d,1H,J1-2=6,1Hz,H-1); 4,47 (qAB,2H,J=16,5Hz,J=3,3Hz,ArOCH₂CO) ; 4,32 (dd,1H,J=10,5Hz,J=8,8Hz,H-3a(A)) ; 4,29 (dAB,1H,J=16,5Hz,ArOCHBCO) ; 4,14 (t,1H,J=8,2Hz,H-3a(B)) ; 4,12 (dd,1H,J=4,78Hz,J=10,4Hz,H-6"A) ; 4,04 (dAB,1H,J=16,4Hz,ArOCHACO) ; 3,48 (s,6H,OCH₃) ; 3,51 (t,1H,J=10,1Hz,H-6"B); 3,44 (t,J=4"-3"=9,4Hz,H-4") ; 3,35 (ddd,1H,J5"-4"=9,6Hz,J5"-6"=4,7Hz,H-5"); 3,08 (dd,1H,J=5,2Hz,J-l4,1Hz,H-2); 2,76 (m,1H,J=10,9Hz,J=3,1Hz,H-3) ; 1,26 (d,3H,J=5,OHz,CH₃).

2ème stade : Hydrogénolyse de la position 4'

Une solution de 1,5 g de l'intermédiaire, issu du 1er stade, dans 15 ml d'acétate d'éthyle distillé, contenant 0,6 g de Pd/c à 10 % est hydrogénée sous 3,45 bars (50 psi) à température ambiante pendant 24 h sous bonne agitation. Après contrôle CCM (éluant CH₂Cl₂MeOH : 95-5), le catalyseur est filtré, le filtrat est évaporé sous pression réduite et chromatographie sur colonne de silice (éluant CH₂Cl₂-MeOH : 97-3). Le produit (940 mg) est obtenu sous forme de cristaux F = 16 °C, Rdt = 85 %. Les caractéristiques physiques sont les suivantes :
IR (Nujol, v, cm⁻¹) : 3400, 1755, 1600, 1500, 1200, 1080.
RMN (400MHz, CDCl₃) δ ppm : 6,93-6,61 (m,6H,OArO) ; 6,60 (s,1H,H-5) ; 6,55 (d,2H,J=9,OHz,OArO); 6,41 (s,1H,H-8) ; 6,15 (s,2H,H-2',H-6') ; 5,85 (s,1H,OCHAO) ; 5,66 (s,1H,OCHBO) ; 5,37 (s,1H,ArOH) ; 5,27 (t,1H,J3"-2"=9,4Hz,H-3"); 5,01 (s,1H,OH) ; 4,95 (dd,1H,J2"-1"=7,8Hz,J2"-3"=9,2Hz,H-2"); 4,81 (d,1H,J=7,8Hz,H-1"); 4,75 (d,1H,J4-3=3,3Hz,H-4); 4,62 (q,1H,J7"-CH₃=4,9Hz,H-7"); 4,46 (qAB,2H,J=16,5Hz,J=7,4Hz,ArOCH₂CO) ; 4,40 (d,1H,J=5Hz,H-1) ; 4,30 (dd,1H,J=10,5Hz,J=8,7Hz,H-3a(A)) ; 4,25 (dAB,1HJ=16,4Hz,ArOCHACO); 4,14 (m,2H,H-6"A,H-3a(B)); 4,01 (dAB,1H,J=16,4Hz,ArOCHB CO) ; 3,67 (s,6H,OCH₃) ; 3,52 (t,1H,J=10,2Hz,H-6"B); 3,44 (t,1H,J4"-3"=9,4Hz,H-4") ; 3,35 (ddd,1H,J5"-4"=9,5Hz,J5"-6"=4,7Hz) ; 3,0 (dd,1H,J2-3=14,OHz,J2-1=5,2Hz,H-2) ; 2,79 (m,1H,H-3) ; 1,27 (d,3H,JCH₃-7"=4,97Hz,CH₃).

| Anal. C₄₅H₄₄O₁₉ | | |
|---|---|---|
| | C | H |
| Calc. % | 60,81 | 4,99 |
| Tr. % | 60,23 | 5,06 |

### Exemple 21

### 4'-déméthyl-4-O-[2,3-bis(4-benzyloxyphénoxy acétyl)-4,6-éthylidène-glucosyl]épipodophyllotoxine.

Une solution de 0,72 g de 4'-benzyloxy carbonyl 4'-déméthyl 4-O-[2,3-bis(4-benzyloxyphénoxyacétyl) 4,6-éthylidène glucosyl] épipodophyllotoxine issu du 1er stade de l'exemple 20 dans 10 ml d'un mélange d'éthanol-acétone (1-1) contenant 0,14 g de catalyseur Pd/C à 10 % est hydrogénée à température ambiante atmosphérique pendant 1 h. Après contrôle en CCM (éluant CH₂Cl₂-MeOH : 97-3), le catalyseur est filtré et le filtrat est évaporé sous pression réduite. Le produit recherché est purifié par chromatographie sur silice (éluant CH₂Cl₃-MeOH : 99-1) et obtenu avec un rendement de 38 %. F = 159 °C.

Les caractéristiques physiques sont les suivantes :
IR (Nujol, v (cm⁻¹)) : 3440, 1750, 1590, 1490, 1200, 1075, 710, 690.
RMN-¹H (400MHz, CDCl₃) δ ppm : 7,30-7,20 (m,10H,Ph) ; 6,79-6,70 (m,6H,OArO) ; 6,68 (s,1H,H-5) ; 6,60 (dm,J=9,OHz,OArO) ; 6,38 (s,2H,H-2',H-6') ; 5,80 (d,1H,J=0,75Hz,OCHAO) ; 5,56 (d,1H,J=0,75Hz,OCHBO) ; 5,33 (s,1H,PhOH-4') ; 5,29 (s,1H,J3"-2"=9,2H₃,J3"-4"=9,4Hz,H-3") ; 4,98 (dd,1H,J2"-1"=7,9Hz,J2"-3"=9,2Hz,H-2") ; 4,84 (d,1H,J=7,6Hz,H-1") ; 4,83 (s,2H,PhCH₂) ; 4,80 (qAB,2H,J=11,6Hz,J=5,OHz,PhCH₂OArO) ; 4,75 (d,1H,J4-3=3,4Hz,H-4) ; 4,61 (q,1H,J7"-CH₃-4,9Hz,H-7"); 4,47 (qAB,2H,J=16,5Hz, J=3,4Hz,ArOCH₂CO) ; 4,45 (d,H,J=7Hz,H-1) ; 4,31 (dd,1H,J=8,96Hz,H-3a(A)); 4,30 (dAB,1H,J=16,3Hz,ArOCHACO); 4,13 (t,1H,jAB=8,1Hz,H-3a(B)) ; 4,125 (dd,1H,JAB=10,6Hz,J6"-5"=4,7Hz,H-6"A); 4,05 (dAB,1H,J=16,4Hz,ArOCHB CO) ; 3,67 (s,6H,OCH₃) ; 3,51 (t,1H,JAB=l0,1Hz,H-6"B); 3,44 (t,1H,J4"-5"=J4"-3"=9,4Hz,H-4") ; 3,35 (ddd,1H,J5"-4"=9,4Hz,J5"-6"=4,7Hz,H-5") ; 3,06 (dd,1H,J2-3=14Hz,J2-1=5,1Hz,H-) ; 2,79 (m,1H,H-3) ; 1,265 (d,3H,JCH₃-7"=4,96Hz,CH₃).

| Anal. C₅₉H₅₇O₁₉ | | |
|---|---|---|
| | C | H |
| Calc. % | 66,22 | 5,37 |
| Tr. % | 66,26 | 5,32 |

Les composés de formule I ci-après ont été préparés selon le même mode opératoire que les exemples précédents :
4'-déméthyl-4-O-[2,3-bis(4-méthyl phénoxyacétyl)-4,6-éthlidène-glucosyl]-épipodophyllotoxine (PF = 130°C, CC" (SiO₂) : Hept-AcOEt (40-60) Rf = 0,35) ;
4'-déméthyl-4-O-[2,3-bis(3, 4, 5-triméthoxy-phénoxyacétyl)4,6-éthylidène-glucosyl]-épipodophyllotoxine (PF = 234°C, CCM (SiO₂) : Hept-AcOEt (30-70) Rf = 0,2) ;
4'-déméthyl-4-O-[2,3-bis(3,4-méthylènedioxy-phénoxyacétyl)4,6-éthylidène-glucosyl]-épipodophyllotxine (PF : 173°C, CCM (SiO₂) : Hept-AcOEt (30-70) Rf = 0,35) ;
4'-déméthyl-4-O-[2,3-bis(3,4-diméthoxy-phenoxyacétyl)-4,6-éthylidène-glucosyl]-épipodophyllotoxine (PF = 194°C, CCM (SiO₂) : CH2Cl2-MeOH (95-5) Rf = 0,4) ;
4'-déméthyl-4-O-[2,3-bis(4-trifluorométhoxy-phénoxyacétyl)4,6-éthylidène-glucosyl]-epipodophyllotoxine (PF = -116°C, CCM (SiO₂) : Hept-AcOEt (50-50) Rf = 0,2) ;
4'-(3,4, 5-triméthoxy-phenoxyacétyl)-4'-déméthyl-4-O-[2,3-bis (3,4,5 -triméthoxy-phénoxyacétyl)-4,6-éthylidène-glucosyl]épipodophyllotoxine (PF = 130°C, CCM (SiO₂) : Hept-AcOEt (50-50) Rf = 0,4) ;
4'-(4 -trifluorométhoxy-phénoxyacétyl)-4'-déméthyl-4-O-[2,3-bis (4-trifluorométhoxy-phénoxyacétyl)-4,6-éthylidène-glucosyl]-épipodophyllotoxine (PF = >260°C, CCM (SiO₂) : Hept-AcOEt (30-70) Rf = 0,4) ;
4'-(4-phényl-phénoxyacétyl )-4'-déméthyl-4-O-[2,3 bis (4-phényl-pénoxyacétyl)4,6-éthylidène-glucosyl]epipodophyllotoxine (PF = 130°C, CCM (SiO₂) : Hept-AcOEt (30-70) Rf = 0,5) ;
4'-(2-naphtoxyacétyl)-4'-déméthyl-4-O-[2,3-bis (2-naphtoxyacetyl)-4,6-éthylidène-glucosyl]-épipodophyllotoxine (PF = 138°C, CCM (SiO₂) : Hept-AcOEt (30-70) Rf = 0,35) ;
4'-(1-naphtoxyacétyl)-4'-déméthyl-4-O-[2,3-bis(1-naphtoxyacétyl)4,6-éthylidène-glucosyl]-épipodophyllotoxine (PF = 125°C, CCM (SiO₂) : Hept-AcOEt (50-50) Pf = 0,5) ;
4'-(cyclohexyloxyacétyl)-4'-déméthyl-4-O-[2,3-bis(cyclohexyloxyacétyl)4,6-éthylidène-glucosyl]-épipodophyllotoxine, (PF = 160°C, CCM (SiO₂) : Hept-AcOEt (50-50) Rf = 0,45) ;
4'-(4-méthyl-phénoxyacétyl)-4'-déméthyl-4-O-[2,3-bis(4-méthyl-phénoxyacétyl)-4,6-éthylidène-glucosyl]épipodophyllotoxine (PF = 151°C, CCM (SiO₂) : Hept-AcOEt (50-50) Rf = 0,4) ;
4'-(3,4-diméthoxy-phénoxyacétyl)-4'-déméthyl-4-O-[2,3-bis (3,4-diméthoxy-phénoxyacétyl)-4,6-éthylidèneglucosyl]épipodophyllotoxine (PF = 158°C, CCM (SiO₂) : Hept-AcOEt (50-50) Rf = 0,27) ;
4'-(3,4-méthylènedioxy-phénoxyacétyl)-4'déméthyl-4-O-[2,3-bis (3,4-méthylènedioxy-phénoxyacétyl)-4,6-éthylidèneglucosyl]épipodophyllotoxine (PF = 158°C, CCM (SiO2) : Hept-AcOEt (20-80) Rf = 0,26) ;
avec Hept pour l'heptane, AcOEt pour l'acétate d'éthyle et CH₂Cl₂ pour le chlorure de méthylène.

### EXPERIMENTATION BIOLOGIOUE

Les molécules ont été testées in vitro et in vivo et ont montré l'intérêt en tant qu'agent anticancéreux dans les tests suivants.

Le test colorimétrique au MTT (appliqué selon la procédure décrite par le NCI) a permis d'évaluer in vitro le pouvoir anti-tumoral desdites molécules sur 6 lignées cellulaires provenant de l'ATCC et incluant des modèles de cancers du sein, de la vessie, du poumon, et du colon.

In vivo, deux tests ont été utilisés, à savoir la leucémie P388 et l'adénocarcinome MXT. Les cellules leucémiques furent injectées i.p. aux animaux au jour J0. Ceux-ci furent ensuite traités par les molécules aux jours J1, J2, J3 et J4. L'adénocarcinome MXT fut transplanté s.c. au jour JO. Les animaux furent traités aux jours J17, J19, J21, J24, J26, J28, J31, J33 et J35. L'indice "T/C" fut évalué pour ces deux modèles murins. Cet indice représente le rapport entre la médiane de survie des animaux traités (T) par rapport à celle des animaux contrôles (C). Cet indice T/C est exprimé en % et lorsque sa valeur dépasse 130 %, la molécule étudiée est définie comme montrant une activité anti-tumorale significative. Lorsque la valeur de T/C dépasse 300 %, on exprime un second indice, LS qui représente le pourcentage d'animaux "Long Survivants" à J30. Il n'est calculé que pour le modèle P388 car le modèle MXT, est trop agressif et ne permet pas l'obtention de tels animaux LS. Chaque groupe expérimental comportait 10 animaux.

Les résultats sont présentés dans le tableau suivant :

| EXEMPLES IN VIVO | | |
|---|---|---|
| | P388* T/C% (LS%) | MXT T/C% |
| Etoposide | 65(O) | 71* |
| 17 | >300(40) | 155* |
| 1 | >300(90) | 139** |
| 18 | >300(100) | 161** |
| 3 | >300(60) | 105* |
| 19 | >300(90) | 160** |
| 14 | >300(60) | 138** |
| 16 | >300(80) | 107** |
| 20 | >300(90) | 136** |

| | | |
|---|---|---|
| * 4 traitements à 40 mg/kg | | |
| ** 9 traitements à 40 mg/kg | | |

Ces produits sont très intéressants par rapport aux produits connus actuellement car ils présentent outre une activité sur les différentes formes de cancer que sont en particulier les carcinomes embryonnaires du testicule, les sarcomes conjonctifs, tumeurs pédiatriques, cancers bronchiques à petites cellules, lymphomes hodgkimiens et non hodgkimiens, leucémies aiguës, cancers du sein, une activité sur les cancers du poumon non à petites cellules et cancers colorectaux où la thérapeutique fait défaut actuellement.

La présente invention concerne donc également les composés de formule générale I, y compris les composés décrits dans l'état de la technique comme intermédiaires de synthèse et exclus par le disclaimer, à titre de médicament, ainsi que leur utilisation pour la préparation d'un médicament destiné au traitement anticancéreux, en particulier les carcinomes embryonnaires du testicule, les sarcomes conjonctifs, les tumeurs pédiatriques, les sarcomes conjonctifs, les tumeurs pédiatriques, les cancers bronchiques à petites cellules, les lymphomes malins, hodgkimiens et non hodgkimiens, les leucémies aigues, les choriocarcinomes placentaires, les adénomes carcinomes mammaires, les cancers colorectaux et les cancers du poumon non à petites cellules.

Elle concerne aussi une composition pharmaceutique comprenant au moins un composé de formule générale I, y compris les composés décrits dans l'état de la technique comme intermédiaires de synthèse et exclus par le disclaimer, et un excipient approprié.

Les compositions pharmaceutiques peuvent être sous une forme appropriée pour l'administration par voie injectable en perfusion ou orale sous forme de capsule de gélules, comprimés, à la posologie de 20 à 200 mg/m² en injectable par 24 h et de 50 à 400 mg/m² par 24 h pour la forme orale.

## Revendications

1. Composé de formule générale I : 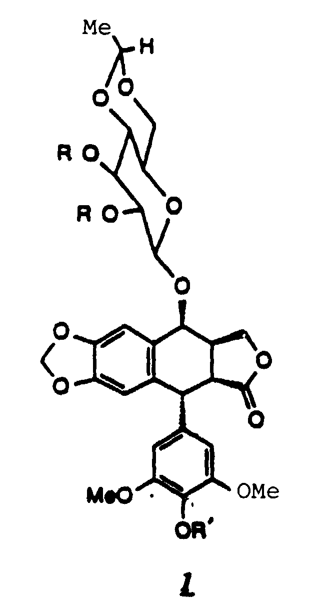 dans laquelle
R' représente un atome d'hydrogène ou un radical R, et
R représente un groupe de formule 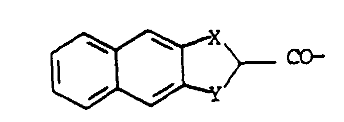 ou un reste acyle de formule
A - Z - CH₂ - CO -
dans lesquelles,
X, Y et Z représentent indépendamment un atome d'oxygène ou un atome de soufre, et
A représente
- un radical alkyle, linéaire ou ramifié en C₁-C₄,
- un radical cycloalkyle en C₃-C₆,
- un radical aryle, en particulier choisi parmi les radicaux phényle, phényle-alkyle, linéraire ou ramifié en C₁-C₄, naphtyle, et ces mêmes radicaux substitués par un à trois substituants, choisis parmi les radicaux hydroxy, alcoxy, linéaire ou ramifié en C₁-C₄ éventuellement perhalogéné avec des atomes de chlore ou de fluor, benzyloxy, phényle, alkyle linéaire ou ramifié en C₁-C₄, et les atomes d'halogène, en particulier le chlore ou le fluor,
et ses sels physiologiquement acceptables,
à la condition que lorsque R' représente un radical R, R est choisi parmi les radicaux 4-fluorophénoxyacétyle, 4-benzyloxy-phénoxyacétyle, 4-hydroxy-phénoxy-acétyle, 4-méthyl-phénoxy-acétyle, 3,4,5-triméthoxy-phénoxyacétyle, 3,4-méthylène-dioxyphénoxyacétyle, 2,3-diméthoxy-phénoxy-acétyle, 4-trifluoro-méthoxy-phénoxyacétyle, 4-phényl-phénoxyacétyle, 1-naphtoxyacétyle, cyclohexylacétyle, 4-méthyl-phénoxyacétyle, 3,4-diméthoxy-phénoxyacétyle.

2. Composé selon la revendication 1, caractérisé en ce que R' représente un atome d'hydrogène.

3. Composé selon l'une des revendications 1 et 2, caractérisé en ce que R est choisi de préférence parmi les radicaux éthoxyacétyle, phénoxyacétyle, 4-méthoxy-phénoxy-acétyle, 2-méthoxyphénoxyacétyle, 4-nitro-phénoxy-acétyle, 2-nitrophénoxyacétyle, 2,4-dichloro-phénoxy-acétyle, 2,4,5-trichloro-phénoxyacétyle, 2,4,6-trichlorophénoxyacétyle, 4-fluoro-phénoxyacétyle, 2-fluorophénoxy-acétyle, 4-hydroxy-phénoxyacétyle, 4-benzyloxyphénoxy-acétyle, 4-méthylphénoxy-acétyle, 3,4,5-triméthoxy-phénoxy-acétyle, 3,4-méthylènedioxyphénoxyacétyle, 2, 3-diméthoxy-phénoxyacétyle, 4-trifluorométhoxy-phénoxyacétyle, 4-phénylphénoxyacétyle, 2-naphtoxyacétyle, 1-naphtoxyacétyle, cyclohexylacétyle, 4-méthylphénoxy-acétyle, 3,4-diméthoxy-phénoxyacétyle.

4. Composé selon l'une des revendications 1 à 3, caractérisé en ce qu'il est choisi parmi :
- 4'-(4-fluorophénoxyacétyl)-4'-déméthyl-4-O-[2,3-bis(4-fluorophénoxyacétyl)-4,-éthylidène glucosyl] épipodphyllotoxine,
- 4'-(4-benzyloxyphénoxyacétyl)-4'-déméthyl-4-O-[2,3-bis(4-benzyloxyphénoxyacétyl)-4,6-éthylidène-glucosyl]-épipodophyllotoxine,
- 4'-(4-hydroxyphénoxyacétyl)-4'-déméthyl-4-O-[2,3-bis(4-hydroxyphénoxyacétyl)-4,6-éthylidène glucosyl]-épipodophyllotoxine,
- 4'-déméthyl 4-O-(2,3-bis-phénoxyacétyl-4,6-éthylidène glucosyl)-épipodophyllotoxine,
- 4'-déméthyl-4-O-[2,3-bis(4-méthoxyphénoxyacétyl)-4,6-éthylidéneglucosyl]épipodophyllotoxine,
- 4'-déméthyl-4-0-[2,3-bis(4-fluorophénoxyacétyl)4,6-éthylidène-glucosyl]-épipodophyllotoxine,
- 4'-déméthyl-4-O-[2,3-bis(4-hydroxyphénoxyacétyl)-4,6-éthylidène-glucosyl]épipodophyllotoxine,
- 4"-déméthyl-4-O-[2,3-bis(4-benzyloxyphénoxyacétyl)-4,6-éthylidène-glucosyl]-épipodophyllotoxine.
- 4'-déméthyl-4-O-[2,3-bis(4-méthyl-phénoxyacétyl)4,6-éthlidène-glucosyl]-épipodophyllotoxine);
- 4 '-déméthyl-4-O-[2,3-bis(3, 4, 5-triméthoxyphénoxyacétyl)-4,6-éthylidène-glucosyl]épipodophyllotoxine
- 4'-déméthyl-4-O-[2,3-bis(3,4-méthylènedioxyphénoxyacétyl)-4,6-éthylidène-glucosyl]épipodophyllotxine
- 4'-déméthyl-4-O-[2,3-bis(3,4-dméthoxy-phénoxyacétyl)4,6-éthylidène-glucosyl]-épipodophyllotoxine
- 4'-déméthyl-4-O-[2,3-bis(4-trifluorométhoxyphénoxyacétyl)-4,6-éthylidène-glucosyl]épipodophyllotoxine
- 4'-(3,4,5-triméthoxy-phenoxyacétyl)-4'-déméthyl-4-O-[2,3-bis (3,4,5-triméthoxy-phénoxyacétyl)4,6-éthylidène-glucosyl]-épipodophyllotoxine
- 4'-(4-trifluorométhoxy-phénoxyacétyl)-4'-déméthyl-4-O-[2,3-bis (4-trifluorométhoxyphénoxyacétyl)-4,6-éthylidène-glucosyl]épipodophyllotoxine
- 4'-(4-phényl-phénoxyacétyl)-4'-déméthyl-4-O-[2,3 bis (4-phényl-pénoxyacétyl)4,6-éthylidène-glucosyl]-epipodophyllotoxine
- 4'-(1-naphtoxyacétyl)-4'-déméthyl-4-O-[2,3-bis(1-naphtoxyacétyl)-4,6-éthylidène-glucosyl]épipodophyllotoxine
- 4'-(cyclohexyloxyacétyl)-4'-démethyl-4-O-[2,3-bis(cyclohexyloxyacétyl)-4,6-éthylidène-glucosyl]épipodophyllotoxine,
- 4'-(4-méthyl-phénoxyacétyl)-4'-déméthyl-4-O-[2,3-bis(4-méthyl-phénoxyacétyl)-4,6-éthylidèneglucosyl]-épipodophyllotoxine
- 4'-(3,4-diméthoxy-phénoxyacétyl)-4'-déméthyl-4-O-[2,3-bis (3,4-diméthoxy-phénoxyacétyl)-4,6-éthylidèneglucosyl]-épipodophyllotoxine
- 4'-(3,4-méthylènedioxy-phénoxyacétyl)-4'déméthyl-4-O-[2,3-bis (3,4-méthylènedioxy-phénoxyacétyl)4,6-éthylidèneglucosyl]-épipodophyllotoxine.

5. Procédé de préparation d'un composé selon l'une des revendication 1 à 4 pour lequel on fait réagir un intermédiaire glycosylé de formule générale 2 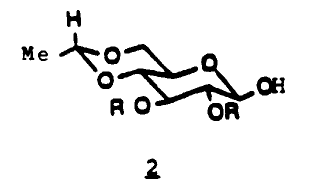 avec un intermédiaire de formule 3 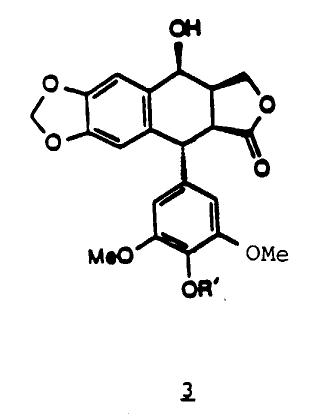 pour lesquelles R et R' sont définis précédemment, et on déprotège éventuellement les fonctions phénol du composé de formule I obtenu.

6. A titre de médicament, le composé de formule générale I : 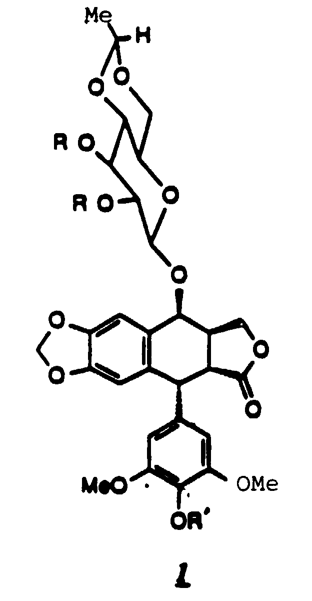 dans laquelle
R' représente un atome d'hydrogène ou un radical R, et
R représente un groupe de formule 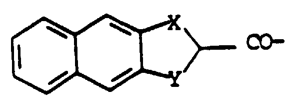 ou un reste acyle de formule
A-Z-CH₂-CO-
dans lesquelles,
X, Y et Z représentent indépendamment un atome d'oxygène ou un atome de soufre, et
A représente
- un radical alkyle, linéaire ou ramifié en C₁-C₄,
- un radical cycloalkyle en C₃-C₆,
- un radical aryle, en particulier choisi parmi les radicaux phényle, phényle-alkyle, linéraire ou ramifié en C₁-C₄, naphtyle, et ces mêmes radicaux substitués par un à trois substituants, choisis parmi les radicaux hydroxy, alcoxy, linéaire ou ramifié en C₁-C₄ éventuellement perhalogéné avec des atomes de chlore ou de fluor, benzyloxy, phényle, alkyle linéaire ou ramifié en C₁-C₄, et les atomes d'halogène, en particulier le chlore ou le fluor,
et ses sels physiologiquement acceptables,

7. Médicament selon la revendication 6, caractérisé en ce que R' représente un atome d'hydrogène.

8. Médicament selon l'une des revendications 6 et 7, caractérisé en ce que R est choisi parmi les éthoxyacétyle, phénoxyacétyle, 4-méthoxy-phénoxyacétyle, 2-méthoxy-phénoxyacétyle, 4-nitro-phénoxyacétyle, 2-nitro-phénoxyacétyle, 2,4-dichlorophénoxy-acétyle, 2,4,5-trichloro-phénoxyacétyle, 2,4,6-trichloro-phénoxyacétyle, 4-fluoro-phénoxyacétyle, 2-fluoro-phénoxy-acétyle, 4-hydroxy-phénoxyacétyle, 4-benzyloxy-phénoxy-acétyle, 4-méthylphénoxyacétyle, 3,4,5-triméthoxy-phénoxy-acétyle, 3,4-méthylènedioxy-phénoxyacétyle, 2,3-diméthoxy-phénoxyacétyle, 4-trifluorométhoxy-phénoxyacétyle, 4-phénylphénoxyacétyle, 2-naphtoxyacétyle, 1-naphtoxyacétyle, cyclohexylacétyle, 4-méthylphénoxy-acétyle, 3,4-diméthoxy-phénoxyacétyle.

9. Médicament selon l'une des revendications 6 à 8, caractérisé en ce qu'il est choisi parmi :
- 4'-phénoxyacétyl-4'-déméthyl-4-O-(2,3-bis-phénoxyacétyl-4,6-éthylidène-glucosyl)-épipodophyllotoxine,
- 4'-(2-méthoxy-phénoxy-acétyl)-4'-déméthyl-4-O-[2,3-bis(2-méthoxyphénoxyacétyl)-4,6-éthylidène-glucosyl]-épipodophyllotoxine,
- 4'-(4-méthoxyphénoxyacétyl)-4'-déméthyl-4-O-[2,3-bis(4-méthoxyphénoxyacétyl)-4,6-éthylidène glucosyl]-épipodophyllotoxine,
- 4'-(2-nitrophénoxyacétyl)-4'-déméthyl-4-O-[2,3-bis(2-nitrophénoxyacétyl) 4,6-éthylidène glucosyl] épipodophyllotoxine,
- 4'-(4-nitrophénoxyacétyl)-4'-déméthyl-4-O-[2,3-bis(4-nitrophénoxyacétyl)-4,6-éthylidène glucosyl] épipodophyllotoxine,
- 4'-(2,4 dichlorophénoxyacétyl)-4'-déméthyl-4-O-[2,3-bis(2,4-dichlorophénoxyacétyl)-4,6-éthylidène-glucosyl]-épipodophyllotoxine.
- 4'-(2,4,5-trichlorophénoxyacétyl)-4'-déméthyl-4-O-[2,3-bis(2,4,5-trichlorophénoxyacétyl)-4,6-éthylidène-glucosyl]-épipodophyllotoxine.
- 4'-(2,4,6-trichlorophénoxyacétyl)-4'-déméthyl 4-O-[2,3-bis(trichlorophénoxyacétyl)-4,6-éthylidène glucosyl] épipodophyllotoxine,
- 4'-(2-fluorophénoxyacétyl)-4'-déméthyl-4-O-[2,3-bis(2-fluorophénoxyacétyl)-4,-éthylidène glucosyl]-épipodophyllotoxine,
- 4'-éthoxyacétyl-4'-déméthyl 4-O-(2,3-bis-éthoxy acétyl-4,6-éthylidène-glucosyl) épipodophyllotoxine.,
- 4'-[2,2-(2,3-naphtylidène-dioxy)-acétyl]-4'-déméthyl-4-O-[2,3-bis[2,2(2,3-naphtylidène dioxy)-acétyl]-4,6-éthylidène-glucosyl]épipodophyllotoxine,
- 4'-benzylthioacétyl-4'-déméthyl-4-O-[2,3-bis benzylthioacétyl-4,6-éthylidène-glucosyl]épipodophyllotoxine,
- 4'-phénylthioacétyl-4'-déméthyl-4-O-[2,3-bis-phénylthioacétyl-4,6-éthylidène-glucosyl]-épipodophyllotoxine,
- 4'-(4-fluorophénoxyacétyl)-4'-déméthyl-4-O-[2,3-bis(4-fluorophénoxyacétyl)-4,-éthylidène glucosyl] épipodphyllotoxine,
- 4'-(4-benzyloxyphénoxyacétyl)-4'-déméthyl-4-O-[2,3-bis(4-benzyloxyphénoxyacétyl)-4,6-éthylidène-glucosyl]-épipodophyllotoxine,
- 4'-(4-hydroxyphénoxyacétyl)-4'-déméthyl-4-O-[2,3-bis(4-hydroxyphénoxyacétyl)-4,6-éthylidène glucosyl]-épipodophyllotoxine,
- 4'-déméthyl 4-O-(2,3-bis-phénoxyacétyl-4,6-éthylidène glucosyl)-épipodophyllotoxine,
- 4'-déméthyl-4-O-[2,3-bis(4-méthoxyphénoxyacétyl)-4,6-éthylidèneglucosyl]épipodophyllotoxine,
- 4'-déméthyl-4-O-[2,3-bis(4-fluorophénoxyacétyl)4,6-éthylidène-glucosyl]-épipodophyllotoxine,
- 4'-déméthyl-4-O-[2,3-bis(4-hydroxyphénoxyacétyl)-4,6-éthylidène-glucosyl]épipodophyllotoxine,
- 4"-déméthyl-4-O-[2,3-bis(4-benzyloxyphénoxyacétyl)-4,6-éthylidène-glucosyl]-épipodophyllotoxine.
- 4'-déméthyl-4-O-[2,3-bis(4-méthyl-phénoxyacétyl)4,6-éthlidène-glucosyl]-épipodophyllotoxine);
- 4'-déméthyl-4-O-[2,3-bis(3, 4, 5-triméthoxyphénoxyacétyl)-4,6-éthylidène-glucosyl]épipodophyllotoxine
- 4'-déméthyl-4-O-[2,3-bis(3,4-méthylènedioxyphénoxyacétyl)-4,6-éthylidène-glucosyl]épipodophyllotxine
- 4'-déméthyl-4-O-[2,3-bis(3,4-diméthoxy-phenoxyacétyl)4,6-éthylidène-glucosyl]-épipodophyllotoxine
- 4'-déméthyl-4-O-[2,3-bis(4-trifluorométhoxyphénoxyacétyl)-4,6-éthylidène-glucosyl]épipodophyllotoxine
- 4'-(3,4,5-triméthoxy-phenoxyacétyl)-4'-déméthyl-4-O-[2,3-bis (3,4,5-triméthoxy-phénoxyacétyl)4,6-éthylidène-glucosyl]-épipodophyllotoxine
- 4'-(4-trifluorométhoxy-phénoxyacétyl)-4'-déméthyl-4-O-[2,3-bis(4-trifluorométhoxyphénoxyacétyl)-4,6-éthylidène-glucosyl]épipodophyllotoxine
- 4'-(4-phényl-phénoxyacétyl)-4'-déméthyl-4-O-[2,3 bis (4-phényl-pénoxyacétyl)4,6-éthylidène-glucosyl]-epipodophyllotoxine
- 4'-(2-naphtoxyacétyl)-4'-déméthyl-4-O-[2,3-bis (2-naphtoxy-acetyl)-4,6-éthylidène-glucosyl]épipodophyllotoxine
- 4 '-(1-naphtoxyacétyl)-4'-déméthyl-4-O-[2,3-bis(1-naphtoxyacétyl)-4,6-éthylidène-glucosyl]épipodophyllotoxine
- 4'-(cyclohexyloxyacétyl)-4'-déméthyl-4-O-[2,3-bis(cyclohexyloxyacétyl)-4,6-éthylidène-glucosyl]-épipodophyllotoxine,
- 4'-(4-méthyl-phénoxyacétyl)-4'-déméthyl-4-O-[2,3-bis(4-méthyl-phénoxyacétyl)-4,6-éthylidène-glucosyl]-épipodophyllotoxine
- 4'-(3,4-diméthoxy-phénoxyacétyl)-4'-déméthyl-4-O-[2,3-bis (3,4-diméthoxy-phénoxyacétyl)-4,6-éthylidèneglucosyl]-épipodophyllotoxine
- 4'-(3,4-méthylènedioxy-phénoxyacétyl)-4'déméthyl-4-O-[2,3-bis (3,4-méthylènedioxy-phénoxyacétyl)-4,6-éthylidèneglucosyl]-épipodophyllotoxine.

10. Composition pharmaceutique caractérisée en ce qu'elle comprend au moins un composé de formule I selon l'une des revendications 6 à 9 et un excipient approprié.

11. Utilisation d'un composé de formule I, selon l'une des revendications 6 à 9, pour la préparation d'un médicament destiné au traitement anticancéreux, en particulier les carcinomes embryonnaires du testicule, les sarcomes conjonctifs, les tumeurs pédiatriques, les cancers bronchiques à petites cellules, les lymphomes malins, hodgkimiens et non hodgkimiens, les leucémies aigues, les choriocarcinomes placentaires, les adénomes carcinomes mammaires, les cancers colorectaux et les cancers du poumon non à petites cellules.

## Patentansprüche

1. Verbindung der allgemeinen Formel I: 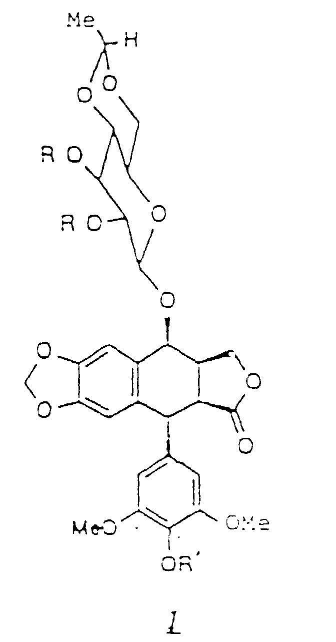 worin bedeuten:
R' ein Wasserstoffatom oder einen Rest R und
R eine Gruppe der Formel 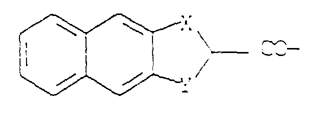 oder einen Acylrest der Formel
A - Z - CH₂ - CO -
worin X, Y und Z unabhängig voneinander für ein Sauerstoff- oder Schwefelatom stehen und A steht für
- einen linearen oder verzweigten C₁-C₄-Alkylrest,
- einen C₃-C₆-Cycloalkylrest,
- einen Arylrest, der insbesondere ausgewählt wird aus den Phenyl-, linearen oder verzweigten C₁-C₄-Alkylphenyl- und Naphthyl-Resten, wobei diese Reste substituiert sind durch 1 bis 3 Substituenten, ausgewählt aus den Hydroxy-, linearen oder verzweigten C₁-C₄-Alkoxy-Resten, die gegebenenfalls perhalogeniert sein können mit Chlor- oder Fluoratomen, Benzyloxy-, Phenyl-, linearen oder verzweigten C₁-C₄-Alkyl-Resten und den Halogenatomen, insbesondere Chlor- oder Fluoratomen,
und ihre physiologisch akzeptablen Salze, mit der Maßgabe, daß dann, wenn R' für einen Rest R steht, R ausgewählt wird unter den 4-Fluorophenoxyacetyl-,4-Benzyloxyphenoxyacetyl-, 4-Hydroxyphenoxyacetyl-, 4-Methyl-phenoxyacetyl-, 3,4,5-Trimethoxy-phenoxyacetyl-, 3,4-Methylendioxyphenoxyacetyl-, 2,3-Dimethoxy-phenoxyacetyl-, 4-Trifluoro-methoxyphenoxyacetyl-, 4-Phenylphenoxyacetyl-, 1-Naphthoxyacetyl-, Cyclohexylacetyl-, 4-Methylphenoxyacetyl- und 3,4-Dimethoxyphenoxyacetyl-Resten.

2. Verbindung nach Anspruch 1, dadurch gekennzeichnet, daß R' für ein Wasserstoffatom steht.

3. Verbindung nach einem der Ansprüche 1 und 2, dadurch gekennzeichnet, daß R vorzugsweise ausgewählt wird unter den Ethoxyacetyl-, Phenoxyacetyl-, 4-Methoxy-phenoxyacetyl-, 2-Methoxyphenoxyacetyl-, 4-Nitro-phenoxyacetyl-, 2-Nitrophenoxyacetyl-, 2,4-Dichloro-phenoxyacetyl-, 2,4,5-Trichlorophenoxyacetyl-, 2,4,6-Trichlorophenoxyacetyl-, 4-Fluoro-phenoxyacetyl-, 2-Fluorophenoxyacetyl-, 4-Hydroxy-phenoxyacetyl-, 4-Benzyloxy-phenoxyacetyl-, 4-Methylphenoxyacetyl-, 3,4,5-Trimethoxy-phenoxyacetyl-, 3,4-Methylendioxy-phenoxyacetyl-, 2,3-Dimethoxyphenoxyacetyl-, 4-Trifluoromethoxyphenoxyacetyl-, 4-Phenylphenoxyacetyl-, 2-Naphthoxyacetyl-, 1-Naphthoxyacetyl-, Cyclohexylacetyl-, 4-Methylphenoxyacetyl- und 3,4-Dimethoxy-phenoxyacetyl-Resten.

4. Verbindung nach einem der Ansprüche 1 bis 3, dadurch gekennzeichnet, daß sie ausgewählt wird aus der Gruppe:
- 4'-(4-Fluorophenoxyacetyl)-4'-demethyl-4-O-[2, 3-bis(4-fluorophenoxyacetyl)-4-ethylidenglucosyl]epipodophyllotoxin,
- 4'-(4-Benzyloxyphenoxyacetyl )-4'-demethyl-4-O-[2, 3-bis(4-benzyloxyphenoxyacetyl)-4,6-ethylidenglucosyl]epipodophyllotoxin,
- 4'-(4-Hydroxyphenoxyacetyl)-4'-demethyl-4-O-[2,3-bis(4-hydroxyphenoxyacetyl)-4,6-ethylidenglucosyl]epipodophyllotoxin,
- 4'-Demethyl-4-O-[2,3-bisphenoxyacetyl)-4,6-ethylidenglucosyl]epipodophyllotoxin,
- 4'-Demethyl-4-O-[2,3-bis(4-methoxyphenoxyacetyl)-4,6-ethylidenglucosyl]-epipodophyllotoxin,
- 4'-Demethyl-4-O-[2,3-bis(4-fluorophenoxyacetyl)-4,6-ethylidenglucosyl]epipodophyllotoxin,
- 4'-Demethyl-4-O-[2,3-bis(4-hydroxyphenoxyacetyl)-4,6-ethylidenglucosyl]-epipodophyllotoxin,
- 4"-Demethyl-4-O-[2,3-bis(4-benzyloxyphenoxyacetyl)-4,6-ethylidenglucosyl]-epipodophyllotoxin,
- 4'-Demethyl-4-O-[2,3-bis(4-methylphenoxyacetyl)-4,6-ethylidenglucosyl]-epipodophyllotoxin,
- 4'-Demethyl-4-O-[2,3-bis(3,4,5-trimethoxyphenoxyacetyl)-4,6-ethylidenglucosyl]-epipodophyllotoxin,
- 4'-Demethyl-4-O-[2,3-bis(3,4-methylendioxyphenoxyacetyl)-4,6-ethylidenglucosyl]-epipodophyllotoxin,
- 4'-Demethyl-4-O-[2,3-bis(3,4-dimethoxyphenoxyacetyl)-4,6-ethylidenglucosyl]-epipodophyllotoxin,
- 4'-Demethyl-4-O-[2,3-bis(4-trifluoromethoxyphenoxyacetyl)-4,6-ethylidenglucosyl]-epipodophyllotoxin,
- 4'-(3,4,5-Trimethoxyphenoxyacetyl)-4'-demethyl-4-O-[2,3-bis(3,4,5-trimethoxyphenoxyacetyl)-4,6-ethylidenglucosyl]-epipodophyllotoxin,
- 4'-(4-Trifluoromethoxyphenoxyacetyl)-4'-demethyl-4-O-[2,3-bis(4-trifluoromethoxyphenoxyacetyl)-4,6-ethylidenglucosyl]-epipodophyllotoxin,
- 4'-(4-Phenylphenoxyacetyl)-4'-demethyl-4-O-[2,3-bis(4-phenylphenoxyacetyl)-4,6-ethylidenglucosyl]-epipodophyllotoxin,
- 4'-(1-Naphthoxyacetyl)-4'-demethyl-4-O-[2,3-bis(1-naphthoxyacetyl)-4,6-ethylidenglucosyl]-epipodophyllotoxin,
- 4'-(Cyclohexyloxyacetyl)-4'-demethyl-4-O-[2,3-bis(cyclohexyloxyacetyl)-4,6-ethylidenglucosyl]-epipodophyllotoxin,
- 4'-(4-Methylphenoxyacetyl)-4'-demethyl-4-O-[2,3-bis(4-methylphenoxyacetyl)-4,6-ethylidenglucosyl]-epipodophyllotoxin,
- 4'-(3,4-Dimethoxyphenoxyacetyl)-4'-demethyl-4-O-[2, 3-bis(3,4-(3,4-dimethoxyphenoxyacetyl)-4,6-ethylidenglucosyl]-epipodphyllotoxin,
- 4'-(3,4-Methylendioxyphenoxyacetyl)-4'-demethyl-4-O-[2,3-bis(3,4-methylendioxyphenoxyacetyl)-4,6-ethylidenglucosyl]-epipodphyllotoxin.

5. Verfahren zur Herstellung einer Verbindung nach einem der Ansprüche 1 bis 4, bei dem man ein glycosyliertes Zwischenprodukt der allgemeinen Formel (2) 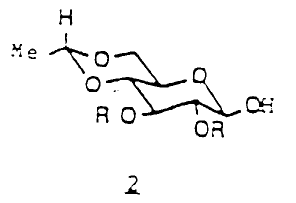 mit einem Zwischenprodukt der Formel (3) reagieren läßt 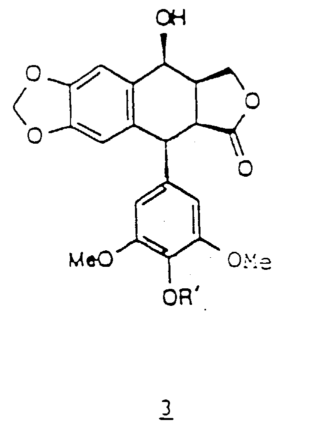 worin R und R' wie oben definiert sind, und
gegebenenfalls die Phenol-Funktionen der erhaltenen Verbindung der Formel (I) von Schutzgruppen befreit.

6. Als Arzneimittel die Verbindung der allgemeinen Formel (I) 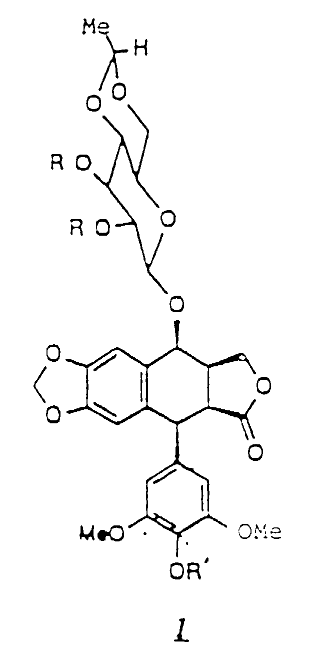 worin bedeuten:
R' ein Wasserstoffatom oder einen Rest R und
R eine Gruppe der Formel 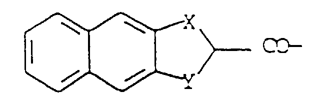 oder einen Acylrest der Formel
A-Z-CH₂-CO-
worin X, Y und Z unabhängig voneinander für ein Sauerstoff- oder Schwefelatom stehen und A steht für
- einen linearen oder verzweigten C₁-C₄-Alkylrest,
- einen C₃-C₆-Cycloalkylrest,
- einen Arylrest, der insbesondere ausgewählt wird aus den Phenyl-, linearen oder verzweigten C₁-C₄-Alkylphenyl- und Naphthyl-Resten, wobei diese Reste substituiert sind durch 1 bis 3 Substituenten, ausgewählt aus den Hydroxy-, linearen oder verzweigten C₁-C₄-Alkoxy-Resten, die gegebenenfalls perhalogeniert sein können mit Chlor- oder Fluoratomen, Benzyloxy-, Phenyl-, linearen oder verzweigten C₁-C₄-Alkyl-Resten und den Halogenatomen, insbesondere Chlor- oder Fluoratomen,
und ihre physiologisch akzeptablen Salze.

7. Arzneimittel nach Anspruch 6, dadurch gekennzeichnet, daß R' für ein Wasserstoffatom steht.

8. Arzneimittel nach einem der Ansprüche 6 und 7, dadurch gekennzeichnet, daß R ausgewählt wird unter den Ethoxyacetyl-, Phenoxyacetyl-, 4-Methoxy-phenoxyacetyl-, 2-Methoxyphenoxyacetyl-, 4-Nitro-phenoxyacetyl-, 2-Nitrophenoxyacetyl-, 2,4-Dichloro-phenoxyacetyl-, 2,4,5-Trichlorophenoxyacetyl-, 2,4,6-Trichlorophenoxyacetyl-, 4-Fluoro-phenoxyacetyl-, 2-Fluorophenoxyacetyl-, 4-Hydroxy-phenoxyacetyl-, 4-Benzyloxy-phenoxyacetyl-, 4-Methylphenoxyacetyl-, 3,4,5-Trimethoxy-phenoxyacetyl-, 3,4-Methylendioxy-phenoxyacetyl-, 2,3-Dimethoxyphenoxyacetyl-, 4-Trifluoromethoxy-phenoxyacetyl-, 4-Phenylphenoxyacetyl-, 2-Naphthoxyacetyl-, 1-Naphthoxyacetyl-, Cyclohexylacetyl-, 4-Methylphenoxyacetyl-, 3,4-Dimethoxy-phenoxyacetyl-Resten.

9. Arzneimittel nach einem der Ansprüche 6 bis 8, dadurch gekennzeichnet, daß es ausgewählt wird aus der Gruppe:
4'-Phenoxyacetyl-4'-demethyl-4-O-(2,3-bis-phenoxyacetyl-4,6-ethylidenglucosyl)-epipodophyllotoxin,
- 4'-(2-Methoxy-phenoxyacetyl)-4'-demethyl-4-O-[2,3-bis-(2-methoxyphenoxyacetyl)-4,6-ethyliden-glucosyl)-epipodophyllotoxin,
- 4'-(4-Methoxy-phenoxyacetyl)-4'-demethyl-4-O-[2,3-bis-(4-methoxyphenoxyacetyl)-4,6-ethyliden-glucosyl)-epipodophyllotoxin,
- 4'-(2-Nitrophenoxyacetyl)-4'-demethyl-4-O-[2,3-bis-(2-nitrophenoxyacetyl)-4,6-ethyliden-glucosyl)-epipodophyllotoxin,
- 4'-(4-Nitrophenoxyacetyl)-4'-demethyl-4-O-[2, 3-bis-(4-nitrophenoxyacetyl)-4,6-ethyliden-glucosyl)-epipodophyllotoxin,
- 4'-(2,4-Dichlorophenoxyacetyl)-4'-demethyl-4-O-[2,3-bis-(2,4-dichlorophenoxyacetyl)-4,6-ethyliden-glucosyl)-epipodophyllotoxin,
- 4'-(2,4,5- Trichlorophenoxyacetyl)-4'-demethyl-4-O-[2,3-bis-(2,4,5-trichlorophenoxyacetyl)-4,6-ethyliden-glucosyl)-epipodophyllotoxin,
- 4'-(2,4,6-Trichlorophenoxyacetyl)-4'-demethyl-4-O-[2,3-bis-(trichlorophenoxyacetyl)-4,6-ethyliden-glucosyl)-epipodophyllotoxin,
- 4'-(2-Fluorophenoxyacetyl)-4'-demethyl-4-O-[2 3-bis-(2-fluorophenoxyacetyl)-4-ethyliden-glucosyl)-epipodophyllotoxin,
- 4'-Ethoxyacetyl-4'-demethyl-4-O-[2,3-bis-ethoxyacetyl-4,6-ethylidenglucosyl)-epipodophyllotoxin,
- 4'-[2,2-(2,3-Naphthylidendioxy)-acetyl)-4'-demethyl-4-O-(2, 3-bis-[2,2(2,3-naphthylidendioxid)acetyl)-4,6-ethyliden-glucosyl]epipodophyllotoxin,
- 4'-Benzylthioacetyl-4'-demethyl-4-O-[2,3-bis-benzylthioacetyl-4,6-ethyliden-glucosyl]-epipodophyllotoxin,
- 4'-Phenylthioacetyl-4'-demethyl-4-O-[2,3-bis-phenylthioacetyl-4,6-ethyliden-glucosyl]-epipodophyllotoxin,
- 4'-(4-Fluorophenoxyacetyl)-4'-demethyl-4-O-[2,3-bis(4-fluorophenoxyacetyl)-4-ethylidenglucosyl]-epipodophyllotoxin,
- 4'-(4-Benzyloxyphenoxyacetyl)-4'-demethyl-4-O-[2, 3-bis(4-benzyloxyphenoxyacetyl)-4,6-ethylidenglucosyl]-epipodophyllotoxin,
- 4'-(4-Hydroxyphenoxyacetyl)-4'-demethyl-4-O-[2, 3-bi s(4-hydroxyphenoxyacetyl)-4,6-ethylidenglucosyl]-epipodophyllotoxin,
- 4'-Demethyl-4-O-(2,3-bisphenoxyacetyl-4,6-ethylidenglucosyl)-epipodophyllotoxin,
- 4'-Demethyl-4-O-[2,3-bis(4-methoxyphenoxyacetyl-4,6-ethylidenglucosyl)-epipodophyllotoxin,
- 4'-Demethyl-4-O-[2,3-bis(4-fluorophenoxyacetyl)-4,6-ethylidenglucosyl)epipodophyllotoxin,
- 4'-Demethyl-4-O-[2,3-bis(4-hydroxyphenoxyacetyl)-4,6-ethylidenglucosyl)-epipodophyllotoxin,
- 4"-Demethyl-4-O-[2,3-bis(4-benzyloxyphenoxyacetyl)-4,6-ethylidenglucosyl)-epipodophyllotoxin,
- 4'-Demethyl-4-O-[2,3-bis(4-methylphenoxyacetyl)-4,6-ethylidenglucosyl]-epipodophyllotoxin,
- 4'-Demethyl-4-O-[2,3-bis(3,4,5-trimethoxyphenoxyacetyl)-4,6-ethylidenglucosyl]-epipodophyllotoxin,
- 4'-Demethyl-4-O-[2,3-bis(3,4-methylendioxyphenoxyacetyl)-4,6-ethylidenglucosyl]-epipodophyllotoxin,
- 4'-Demethyl-4-O-[2,3-bis(3,4-dimethoxyphenoxyacetyl)-4,6-ethylidenglucosyl]-epipodophyllotoxin,
- 4'-Demethyl-4-O-[2,3-bis(4-trifluoromethoxyphenoxyacetyl)-4,6-ethylidenglucosyl]-epipodophyllotoxin,
- 4'-(3,4,5-Trimethoxy-phenoxyacetyl)-4'-demethyl-4-O-[2,3-bis(3,4,5-trimethoxyacetyl)-4,6-ethylidenglucosyl]-epipodophyllotoxin,
- 4'-(4-Trifluoromethoxy phenoxyacetyl)-4'-demethyl-4-O-[2,3-bis(4-trifluoromethoxyphenoxyacetyl)-4,6-ethylidenglucosyl]epipodophyllotoxin,
4'-(4-Phenyl-phenoxyacetyl)-4'-demethyl-4-O-[2, 3-bis(4-phenylphenoxyacetyl)-4,6-ethylidenglucosyl]-epipodophyllotoxin,
- 4'-(2-Naphthoxyacetyl)-4'-demethyl-4-O-[2,3-bis(2-naphthoxyacetyl)-4,6-ethylidenglucosyl]-epipodophyllotoxin,
- 4'-(1-Naphthoxyacetyl)-4'-demethyl-4-O-[2,3-bis(1-naphthoxyacetyl)-4,6-ethylidenglucosyl]-epipodophyllotoxin,
- 4'-(Cyclohexyloxyacetyl)-4'-demethyl-4-O-[2,3-bis(cyclohexyloxyacetyl)4,6-ethylidenglucosyl]-epipodophyllotoxin,
- 4'-(4-Methylphenoxyacetyl)-4'-demethyl-4-O-[2, 3-bis(4-methylphenoxyacetyl)-4,6-ethylidenglucosyl]-epipodophyllotoxin,
- 4'-(3,4-Dimethoxyphenoxyacetyl)-4'-demethyl-4-O-[2,3-bis(3,4-dimethoxy-phenoxyacetyl)-4,6-ethylidenglucosyl]-epipodophyllotoxin,
- 4'-(3,4-Methylendioxyphenoxyacetyl)-4'-demethyl-4-O-[2,3-bis(3,4-methylendioxyphenoxyacetyl)-4,6-ethylidenglucosyl]-epipodophyllotoxin,

10. Pharmazeutische Zusammensetzung, dadurch gekennzeichnet, daß sie mindestens eine Verbindung der Formel (I) nach einem der Ansprüche 6 bis 9 und einen geeigneten Exzipienten enthält.

11. Verwendung einer Verbindung der Formel (I) nach einem der Ansprüche 6 bis 9 zur Herstellung eines Arzneimittels für die Behandlung von Krebs, insbesondere für die Behandlung von embryonalen Hodencarcinomen, Bindegewebssarcomen, pädiatrischen Tumoren, kleinzelligen Bronchialkrebsen, malignen Hodgkin- und Nicht-Hodgkin-Lymphomen, akuten Leukämien, Placentachorio-Carcinomen, Mammaadenom-Carcinomen, Colorectal-Krebsen und nicht-kleinzelligen Lungenkrebsen.

## Claims

1. Compound of general formula 1: 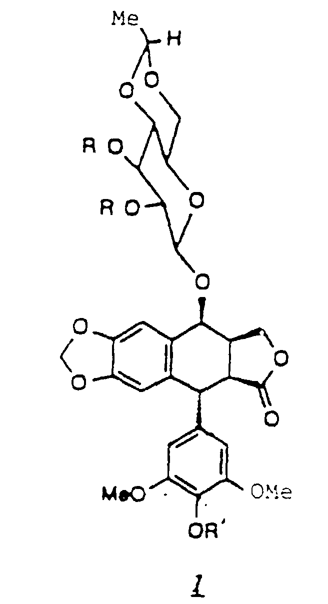 in which
R' represents a hydrogen atom or a radical R,
and
R represents a group of formula 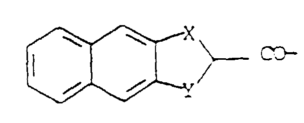
or an acyl residue of formula
A - Z - CH₂ - CO -
in which formulae
X, Y and Z independently represent an oxygen atom or a sulphur atom, and
A represents
- a linear or branched C₁-C₄ alkyl radical,
- a C₃-C₆ cycloalkyl radical,
- an aryl radical chosen especially from phenyl, phenyl(linear or branched C₁-C₄ alkyl) and naphthyl radicals and these same radicals substituted with one to three substituents chosen from hydroxyl radicals, linear or branched C₁-C₄ alkoxy radicals optionally perhalogenated with chlorine or fluorine atoms, benzyloxy, phenyl and linear or branched C₁-C₄ alkyl radicals and halogen atoms, especially chlorine or fluorine,
and its physiologically acceptable salts,
on the condition that, when R' represents a radical R, R is chosen from 4-fluorophenoxyacetyl, 4-benzyloxyphenoxyacetyl, 4-hydroxyphenoxyacetyl, 4-methylphenoxyacetyl, 3,4,5-trimethoxyphenoxyacetyl, 3,4-methylenedioxyphenoxyacetyl, 2,3-dimethoxyphenoxyacetyl, 4-(trifluoromethoxy)phenoxyacetyl, 4-phenylphenoxyacetyl, 1-naphthyloxyacetyl, cyclohexylacetyl, 4-methylphenoxyacetyl and 3,4-dimethoxyphenoxyacetyl radicals.

2. Compound according to Claim 1, characterized in that R' represents a hydrogen atom.

3. Compound according to one of Claims 1 and 2, characterized in that R is preferably chosen from ethoxyacetyl, phenoxyacetyl, 4-methoxyphenoxyacetyl, 2-methoxyphenoxyacetyl, 4-nitrophenoxyacetyl, 2-nitrophenoxyacetyl, 2,4-dichlorophenoxyacetyl, 2,4,5-trichlorophenoxyacetyl, 2,4,6-trichlorophenoxyacetyl, 4-fluorophenoxyacetyl, 2-fluorophenoxyacetyl, 4-hydroxyphenoxyacetyl, 4-benzyloxyphenoxyacetyl, 4-methylphenoxyacetyl, 3,4,5-trimethoxyphenoxyacetyl, 3,4-methylenedioxyphenoxyacetyl, 2,3-dimethoxyphenoxyacetyl, 4-(trifluoromethoxy)phenoxyacetyl, 4-phenylphenoxyacetyl, 2-naphthyloxyacetyl, 1-naphthyloxyacetyl, cyclohexylacetyl, 4-methylphenoxyacetyl and 3,4-dimethoxyphenoxyacetyl radicals.

4. Compound according to one of Claims 1 to 3, characterized in that it is chosen from :
- 4'-(4-fluorophenoxyacetyl)-4"-demethyl-4-O-[2,3-bis(4-fluorophenoxyacetyl)-4,6-ethylideneglucosyl]epipodophyllotoxin,
- 4'-(4-benzyloxyphenoxyacetyl)-4'-demethyl-4-O-[2,3-bis(4-benzyloxyphenoxyacetyl)-4,6-ethylideneglucosyl]epipodophyllotoxin,
- 4'-(4-hydroxyphenoxyacetyl)-4'-demethyl-4-O-[2,3-bis(4-hydroxyphenoxyacetyl)-4,6-ethylideneglucosyl]epipodophyllotoxin,
- 4'-demethyl-4-O-[2,3-bis(phenoxyacetyl)-4,6-ethylideneglucosyl]epipodophyllotoxin,
- 4'-demethyl-4-O-[2,3-bis(4-methoxyphenoxyacetyl)4,6-ethylideneglucosyl]epipodophyllotoxin,
- 4'-demethyl-4-O-[2,3-bis(4-fluorophenoxyacetyl)-4,6-ethylideneglucosyl]epipodophyllotoxin,
- 4'-demethyl-4-O-[2,3-bis(4-hydroxyphenoxyacetyl)4,6-ethylideneglucosyl]epipodophyllotoxin,
- 4'-demethyl-4-O-[2,3-bis(4-benzyloxyphenoxyacetyl)4,6-ethylideneglucosyl]epipodophyllotoxin,
- 4'-demethyl-4-O-[2,3-bis(4-methylphenoxyacetyl)-4,6-ethylideneglucosyl]epipodophyllotoxin),
- 4'-demethyl-4-O-[2,3-bis(3,4,5-trimethoxyphenoxyacetyl)-4,6-ethylideneglucosyl]epipodophyllotoxin,
- 4'-demethyl-4-O-[2,3-bis(3,4-methylenedioxyphenoxyacetyl)-4,6-ethylideneglucosyl]epipodophyllotoxin,
- 4'-demethyl-4-O-[2,3-bis(3,4-dimethoxyphenoxyacetyl)-4,6-ethylideneglucosyl]epipodophyllotoxin,
- 4'-demethyl-4-O-[2,3-bis(4-trifluoromethoxy)phenoxyacetyl)-4,6-ethylideneglucosyl]epipodophyllotoxin,
- 4'-(3,4,5-trimethoxyphenoxyacetyl)-4'-demethyl-4-O-[2,3-bis(3,4,5-trimethoxyphenoxyacetyl)-4,6-ethylideneglucosyl]epipodophyllotoxin,
- 4'-(4-(trifluoromethoxy)phenoxyacetyl)-4' -demethyl-4-O-[2,3-bis(4-(trifluoromethoxy)phenoxyacetyl)-4,6-ethylideneglucosyl]epipodophyllotoxin,
- 4'-(4-phenylphenoxyacetyl)-4'-demethyl-4-O-[2,3-bis(4-phenylphenoxyacetyl)-4,6-ethylideneglucosyl]-epipodophyllotoxin,
- 4'-(1-naphthyloxyacetyl)-4'-demethyl-4-O- [2,3-bis(1-naphthyloxyacetyl)-4,6-ethylideneglucosyl]epipodophyllotoxin,
- 4'-(cyclohexyloxyacetyl)-4'-demethyl-4-O-[2,3-bis(cyclohexyloxyacetyl)-4,6-ethylideneglucosyl]epipodophyllotoxin,
- 4'-(4-methylphenoxyacetyl)-4'-demethyl-4-O-[2,3-bis(4-methylphenoxyacetyl)-4,6-ethylideneglucosyl]epipodophyllotoxin,
- 4'-(4-methylphenoxyacetyl)-4'-demethyl-4-O- [2,3-bis(4-methylphenoxyacetyl)-4,6-ethylideneglucosyl]epipodophyllotoxin,
- 4'-(3,4-dimethoxyphenoxyacetyl)-4'-demethyl-4-O-[2,3-bis(3,4-dimethoxyphenoxyacetyl)-4,6-ethylideneglucosyl]epipodophyllotoxin,
- 4'-(3,4-methylenedioxyphenoxyacetyl)-4'-demethyl-4-O-[2,3-bis(3,4-methylenedioxyphenoxyacetyl)-4,6-ethylideneglucosyl]epipodophyllotoxin.

5. Process for preparing a compound according to one of Claims 1 to 4, for which a glycosyl intermediate of general formula 2 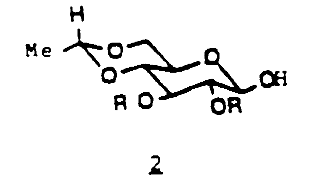 is reacted with an intermediate of formula 3 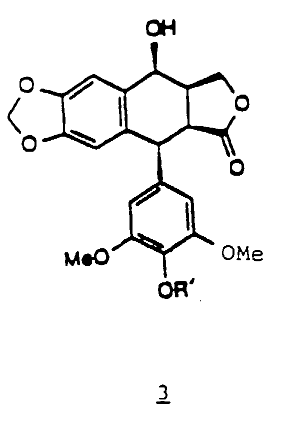 for which R and R' are defined above, and the phenol functions of the compound of formula 1 obtained are, where appropriate, deprotected.

6. As a medicinal product, the compound of general formula 1: 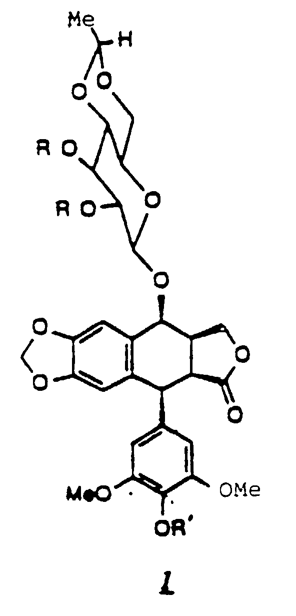 in which
R' represents a hydrogen atom or a radical R,
and
R represents a group of formula 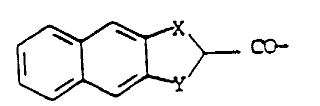 or an acyl residue of formula
A-Z-CH₂-CO-
in which formulae
X, Y and Z independently represent an oxygen atom or a sulphur atom, and
A represents
- a linear or branched C₁-C₄ alkyl radical,
- a C₃-C₆ cycloalkyl radical,
- an aryl radical chosen especially from phenyl, phenyl(linear or branched C₁-C₄ alkyl) and naphthyl radicals and these same radicals substituted with one to three substituents chosen from hydroxyl radicals, linear or branched C₁-C₄ alkoxy radicals optionally perhalogenated with chlorine or fluorine atoms, benzyloxy, phenyl and linear or branched C₁-C₄ alkyl radicals and halogen atoms, especially chlorine or fluorine,
and its physiologically acceptable salts.

7. Medicinal product according to Claim 6, characterized in that R' represents a hydrogen atom.

8. Medicinal product according to one of Claims 6 and 7, characterized in that R is chosen from ethoxyacetyl, phenoxyacetyl, 4-methoxyphenoxyacetyl, 2-methoxyphenoxyacetyl, 4-nitrophenoxyacetyl, 2-nitrophenoxyacetyl, 2,4-dichlorophenoxyacetyl, 2,4,5-trichlorophenoxyacetyl, 2,4,6-trichlorophenoxyacetyl, 4-fluorophenoxyacetyl, 2-fluorophenoxyacetyl, 4-hydroxyphenoxyacetyl, 4-benzyloxyphenoxyacetyl, 4-methylphenoxyacetyl, 3,4,5-trimethoxyphenoxyacetyl, 3,4-methylenedioxyphenoxyacetyl, 2,3-dimethoxyphenoxyacetyl, 4-(trifluoromethoxyphenoxyacetyl, 4-phenylphenoxyacetyl, 2-naphthyloxyacetyl, 1-naphthyloxyacetyl, cyclohexylacetyl, 4-methylphenoxyacetyl and 3,4-dimethoxyphenoxyacetyl radicals.

9. Medicinal product according to one of Claims 6 to 8, characterized in that it is chosen from:
- 4'-phenoxyacetyl-4'-demethyl-4-O-[2,3-bis(phenoxyacetyl)-4,6-ethylideneglucosyl]epipodophyllotoxin,
- 4'-(2-methoxyphenoxyacetyl)-4'-demethyl-4-O-[2,3-bis(2-methoxyphenoxyacetyl)-4,6-ethylideneglucosyl]epipodophyllotoxin,
- 4'-(4-methoxyphenoxyacetyl)-4'-demethyl-4-O-[2,3-bis(4-methoxyphenoxyacetyl)-4,6-ethylideneglucosyl]epipodophyllotoxin,
- 4'-(2-nitrophenoxyacetyl)-4'-demethyl-4-O-[2,3-bis(2-nitrophenoxyacetyl)-4,6-ethylideneglucosyl]epipodophyllotoxin,
- 4'-(4-nitrophenoxyacetyl)-4'-demethyl-4-O-[2,3-bis(4-nitrophenoxyacetyl)-4,6-ethylideneglucosyl]epipodophyllotoxin,
- 4'-(2,4-dichlorophenoxyacetyl)-4-demethyl-4-O-[2,3-bis(2,4-dichlorophenoxyacetyl)-4,6-ethylideneglucosyl]epipodophyllotoxin,
- 4'-(2,4,5-trichlorophenoxyacetyl)-4'-demethyl-4-O-[2,3-bis(2,4,5-trichlorophenoxyacetyl)-4,6-ethylideneglucosyl]epipodophyllotoxin,
- 4'-(2,4,6-trichlorophenoxyacetyl)-4'-demethyl-4-O-[2,3-bis(2,4,6-trichlorophenoxyacetyl)-4,6-ethylideneglucosyl]epipodophyllotoxin,
- 4'-(2-fluorophenoxyacetyl)-4'-demethyl-4-O-[2,3-bis(2-fluorophenoxyacetyl)-4-ethylideneglucosyl]epipodophyllotoxin,
- 4'-ethoxyacetyl-4'-demethyl-4-O-[2,3-bis(ethoxyacetyl)-4,6-ethylideneglucosyl]epipodophyllotoxin,
- 4'-[2,2-(2,3-naphthylidenedioxy)acetyl]-4'-demethyl4-O-[2,3-bis[2,2-(2,3-naphthylidenedioxy)acetyl]4,6-ethylideneglucosyl]epipodophyllotoxin,
- 4'-benzylthioacetyl-4'-demethyl-4-O-[2,3-bis(benzylthioacetyl)-4,6-ethylideneglucosyl]epipodophyllotoxin,
- 4'-phenylthioacetyl-4'-demethyl-4-O-[2,3-bis(phenylthioacetyl)-4,6-ethylideneglucosyl]epipodophyllotoxin,
- 4'-(4-fluorophenoxyacetyl)-4'-demethyl-4-O-[2,3-bis(4-fluorophenoxyacetyl)-4,6-ethylideneglucosyl]epipodphyllotoxin,
- 4'-(4-benzyloxyphenoxyacetyl)-4'-demethyl-4-O-[2,3-bis(4-benzyloxyphenoxyacetyl)-4,6-ethylideneglucosyl]epipodophyllotoxin,
- 4'-(4-hydroxyphenoxyacetyl)-4'-demethyl-4-O-[2,3-bis(4-hydroxyphenoxyacetyl)-4,6-ethylideneglucosyl]epipodophyllotoxin,
- 4'-demethyl-4-O-[2,3-bis(phenoxyacetyl)-4,6-ethylideneglucosyl]epipodophyllotoxin,
- 4'-demethyl-4-O-[2,3-bis(4-methoxyphenoxyacetyl)4,6-ethylideneglucosyl]epipodophyllotoxin,
- 4'-demethyl-4-O-[2,3-bis(4-fluorophenoxyacetyl)-4,6-ethylideneglucosyl]epipodophyllotoxin,
- 4'-demethyl-4-O-[2,3-bis(4-hydroxyphenoxyacetyl)4,6-ethylideneglucosyl]epipodophyllotoxin,
- 4"-demethyl-4-O-[2,3-bis(4-benzyloxyphenoxyacetyl)4,6-ethylideneglucosyl]epipodophyllotoxin,
- 4'-demethyl-4-O-[2,3-bis(4-methylphenoxyacetyl)-4,6-ethylideneglucosyl]epipodophyllotoxin),
- 4'-demethyl-4-O-[2,3-bis(3,4,5-trimethoxyphenoxyacetyl)-4,6-ethylideneglucosyl]epipodophyllotoxin,
- 4'-demethyl-4-O-[2,3-bis(3,4-methylenedioxyphenoxyacetyl)-4,6-ethylideneglucosyl]epipodophyllotoxin,
- 4'-demethyl-4-O-[2,3-bis(3,4-dimethoxyphenoxyacetyl)-4,6-ethylideneglucosyl]epipodophyllotoxin,
- 4'-demethyl-4-O-[2,3-bis(4-trifluoromethoxy)phenoxyacetyl)-4,6-ethylideneglucosyl]epipodophyllotoxin,
- 4'-(3,4,5-trimethoxyphenoxyacetyl)-4'-demethyl-4-O-[2,3-bis(3,4,5-trimethoxyphenoxyacetyl)-4,6-ethylideneglucosyl]epipodophyllotoxin,
- 4'-(4-(trifluoromethoxy)phenoxyacetyl)-4'-demethyl-4-O-[2,3-bis(4-(trifluoromethoxy)phenoxyacetyl)-4,6-ethylideneglucosyl]epipodophyllotoxin,
- 4'-(4-phenylphenoxyacetyl)-4'-demethyl-4-O-[2,3-bis(4-phenylphenoxyacetyl)-4,6-ethylideneglucosyl]epipodophyllotoxin,
- 4'- (2-naphthyloxyacetyl)-4'-demethyl-4-O-[2,3-bis(2-naphthyloxyacetyl)-4,6-ethylideneglucosyl]epipodophyllotoxin,
- 4'-(1-naphthyloxyacetyl)-4'-demethyl-4-O-[2,3-bis(1-naphthyloxyacetyl)-4,6-ethylideneglucosyl]epipodophyllotoxin,
- 4'-(cyclohexyloxyacetyl)-4'-demethyl-4-O- [2,3-bis(cyclohexyloxyacetyl)-4,6-ethylideneglucosyl]epipodophyllotoxin,
- 4'-(4-methylphenoxyacetyl)-4'-demethyl-4-O-[2,3-bis(4-methylphenoxyacetyl)-4,6-ethylideneglucosyl]epipodophyllotoxin,
- 4'-(3,4-dimethoxyphenoxyacetyl)-4'-demethyl-4-O-[2,3-bis(3,4-dimethoxyphenoxyacetyl)-4,6-ethylideneglucosyl]epipodophyllotoxin,
- 4'-(3,4-methylenedioxyphenoxyacetyl)-4'-demethyl-4-O-[2,3-bis(3,4-methylenedioxyphenoxyacetyl)-4,6ethylideneglucosyl]epipodophyllotoxin.

10. Pharmaceutical composition, characterized in that it comprises at least one compound of formula 1 according to one of Claims 6 to 9, and a suitable excipient.

11. Use of a compound of formula 1 according to one of Claims 6 to 9, for the preparation of a medicinal product intended for anticancer treatment, especially embryonic carcinomas of the testicle, connective tissue sarcomas, pediatric tumours, small cell bronchial cancers, Hodgkin's and non-Hodgkin's malignant lymphomas, acute leukaemias, placental choriocarcinomas, mammary adenomes carcinomas, colorectal cancers and non-small cell cancers of the lung.
